# FASCICULE DE BREVET EUROPEEN

(11) **EP 1 535 947 B1**
(45) Date de publication et mention de la délivrance du brevet: **06.01.2010**
(21) Numéro de dépôt: 04292615.4
(22) Date de dépôt: 04.11.2004
(51) Int. Cl.: C08G 77/04, C07F 7/08, A61K 8/89

(54) **Composés diorganopolysiloxaniques à fonction 4,4-diarylbutadiène compositions photoprotectrices les contenant ; utilisations**
4,4-Diarylbutadien enthaltende Diorganopolysiloxan-Derivate, diese enthaltende Lichtschutzzusammensetzungen und deren Verwendungen
Diorganosiloxane derivatives containing a 4,4-diarylbutadiene moiety, sunscreen compositions containing them and their uses

(30) Priorité: 25.11.2003 FR 0350900
(43) Date de publication de la demande: 01.06.2005
(73) Titulaire: L'ORÉAL, 75008 Paris (FR)
(72) Inventeur: Richard, Hervé, 93420 Villepinte (FR)
(74) Mandataire: Miszputen, Laurent

(56) Documents cités:
- EP-A- 0 712 855
- EP-A- 0 845 466
- WO-A-03/027168
- PATENT ABSTRACTS OF JAPAN vol. 2002, no. 06, 4 juin 2002 (2002-06-04) -& JP 2002 053527 A (TAKASAGO INTERNATL CORP), 19 février 2002 (2002-02-19)

## Description

L'invention concerne de nouveaux composés diorganosiloxaniques à fonction 4,4-diarylbutadiène.

L'invention concerne l'utilisation de composés diorganosiloxaniques à fonction 4,4-diarylbutadiène dans des compositions cosmétiques comme agent de filtration des rayons UV-A.

L'invention concerne des compositions photoprotectrices comprenant des composés diorganosiloxaniques à fonction 4,4-diarylbutadiène à titre de filtres solaires actifs dans le domaine des rayonnements UV et plus particulièrement dans de le domaine des rayonnements UV-A.

On sait que les radiations de longueurs d'onde comprises entre 280 nm et 400 nm permettent le brunissement de l'épiderme humain, et que les radiations de longueurs d'onde comprises entre 280 et 320 nm connues sous la dénomination de radiations UV-B, provoquent des érythèmes et des brûlures cutanées qui peuvent nuire au développement du bronzage naturel. Pour ces raisons ainsi que pour des raisons esthétiques, il existe une demande croissante de moyens de contrôle de ce bronzage naturel. Il convient donc de filtrer ce rayonnement UV-B.

On sait également que les rayons UV-A, de longueurs d'onde comprises entre 320 et 400 nm, provoquant le brunissement de la peau, sont susceptibles d'induire une altération de celle-ci, notamment dans le cas d'une peau sensible et/ou continuellement exposée au rayonnement solaire. Les rayons UV-A entraînent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photoallergiques. Ainsi, pour des raisons esthétiques et cosmétiques telles que la conservation de l'élasticité naturelle de la peau, de plus en plus de personnes désirent contrôler l'effet des rayons UV-A sur leur peau. Il est donc souhaitable de filtrer aussi le rayonnement UV-A.

Il existe aujourd'hui un besoin de plus en plus important de rechercher des composés aromatiques efficaces pour la filtration des radiations UV-A et de préférence dans la gamme allant de 340 à 400 nm (gamme dite des UV-A longs ou UVA-I). Outre leur bonne efficacité de photoprotection dans cette gamme, les filtres UV-A recherchés doivent présenter de bonnes propriétés cosmétiques, une bonne solubilité dans les solvants usuels et en particulier dans les corps gras tels que les huiles et les graisses, une bonne résistance à l'eau et à la transpiration (rémanence) ainsi qu'une photostabilité satisfaisante.

Il existe actuellement sur le marché des filtres UV un nombre limité de composés organiques efficaces vis à vis des rayons UV-A et notamment des UV-A longs.

A cet égard, une famille de filtres UV-A particulièrement efficace dans la gamme des UV-A est actuellement constituée par les dérivés du dibenzoylméthane, et notamment le 4-ter-butyl-4'-méthoxydibenzoyl méthane, qui présentent en effet un fort pouvoir d'absorption intrinsèque. Ces dérivés du dibenzoylméthane, qui sont maintenant des produits bien connus en soi à titre de filtres actifs dans les UV-A, sont notamment décrits dans les demandes de brevets français FR-A-2326405 et FR-A-2440933, ainsi que dans la demande de brevet européen EP-A-0114607 ; le 4-ter-butyl-4'-méthoxydibenzoyl méthane est par ailleurs actuellement proposé à la vente sous la dénomination commerciale de "PARSOL 1789" par la Société HOFFMANN LAROCHE.

Malheureusement, il se trouve que les dérivés du dibenzoylméthane sont des produits relativement photosensibles au rayonnement UV-A, c'est-à-dire, plus précisément, qu'ils présentent une fâcheuse tendance à se dégrader plus ou moins rapidement sous l'action de ce dernier. Ainsi, ce manque substantiel de stabilité photochimique des dérivés du dibenzoylméthane face au rayonnement ultraviolet auquel ils sont par nature destinés à être soumis, ne permet pas de garantir une protection constante durant une exposition solaire prolongée, de sorte que des applications répétées à intervalles de temps réguliers et rapprochés doivent être effectuées par l'utilisateur pour obtenir une protection efficace de la peau contre les rayons UV.

Une autre difficulté, indépendante de celle évoquée ci-avant, rencontrée avec les dérivés de dibenzoylméthane est qu'il s'agit de filtres lipophiles présentant la particularité mais aussi le désavantage d'être solides à température ambiante. De ce fait, leur utilisation dans une composition cosmétique antisolaire implique certaines contraintes au niveau de leur formulation et de leur mise en oeuvre, en particulier lorsqu'il s'agit de trouver des solvants permettant de les solubiliser correctement, seuls ou conjointement avec d'autres filtres.

Il existe donc un besoin de rechercher de nouvelles familles de composés aromatiques qui soient efficaces en matière de filtration dans la gamme des UV-A et notamment celle des UVA longs mais qui soient photostables et présentent également une bonne solubilité dans les solvants usuels, de bonnes propriétés cosmétiques ainsi qu'une bonne résistance à l'eau et à la transpiration (rémanence).

On connaît dans l'art antérieur des silicones filtres tels que dans la demande WO03/027168 des silicones à fonction ester cinnnamate, dans la demande EP0712855 des diorganosiloxanes à chaînes courtes linéaires ou cycliques présentant des motifs filtrants à fonction sulfonamide choisis au sein des dérivés de benzylidènecamphre, des benzotriazole, des benzophénones ou benzidazoles ; dans la demande EP845466 des silicones filtres à fonction cinnamamide, benzalmalonamide ou benzalmalonate. On connaît dans la demande JP2002 053527 des composés 4,4-diarylbutadiènes non siliconés.

La Demanderesse a découvert de manière surprenante une nouvelle famille de composés diorganosiloxaniques à fonction 4,4-diarylbutadiène permettant d'atteindre ces objectifs.

Un objet de la présente invention consiste en une nouvelle famille de composés composés diorganosiloxaniques à fonction 4,4-diarylbutadiène de formule générale (1) ou (2) que l'on définira en détail ci-dessous.

L'invention concerne l'utilisation d'au moins un composé diorganosiloxanique à fonction 4,4-diarylbutadiène de formule générale (1) ou (2) que l'on définira en détail ci-dessous, dans la préparation de compositions cosmétiques ou dermatologiques comme agent de filtration des rayons de longueurs d'onde comprises entre 320 et 400 nm.

L'invention concerne des compositions photoprotectrices, comprenant au moins un composé diorganosiloxanique à fonction 4,4-diarylbutadiène de formule générale (1) que l'on définira en détail ci-dessous.

Les composés diorganosiloxaniques à fonction 4,4-diarylbutadiène, conformes à l'invention sont sous forme isolée ou de mélange et correspondent à l'une des formules générales (1) ou (2) suivante : 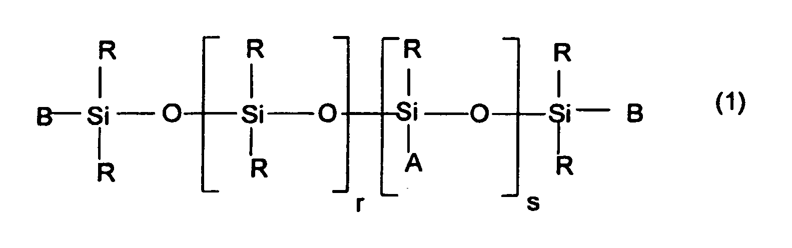 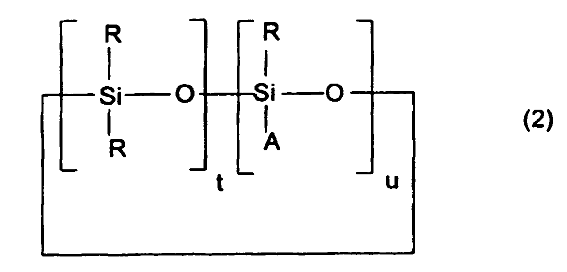 dans lesquelles :
- A désigne un radical de formule (3) suivante : 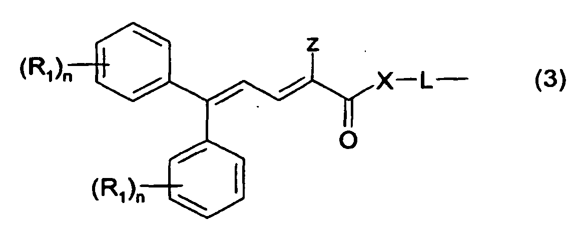
dans laquelle :
- les radicaux R₁ , égaux ou différents, désignent un radical hydroxyle ; un radical alkyle en C₁-C₁₀, linéaire ou ramifié, saturé ou insaturé ; un radical alcoxy en C₁-C₁₀ linéaire ou ramifié, saturé ou insaturé, 2 groupes R₁ adjacents pouvant former ensemble un cycle alkylène dioxy en C₁-C₃ ;
- n est un entier allant de 0 à 2 ;
- le radical Z désigne hydrogène ; -(C=O)OR₂, -(C=O)R₃, -(C=O)NR₄R₅ , -SO₂R₆ -CN ou -(C=O)-X-L- ,
- X désigne -O- ou -NR₄-,
- L est un radical divalent permettant l'accrochage du radical A sur la chaîne siliconée répondant à l'une des formules (a) , (a') ou (a") suivantes :

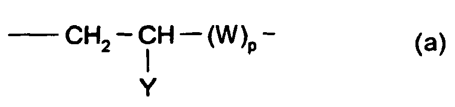

   ―CH=CH-(W)ₚ- (a') 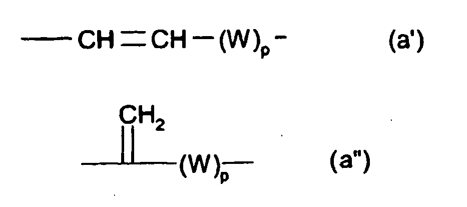
dans lesquelles :
- W est un radical alkylène en C₁-C₆ linéaire ou ramifié, saturé ou insaturé, éventuellement substitué par un radical hydroxyle ou un radical alkyle en C₁-C₈, linéaire ou ramifié, saturé ou insaturé,
- Y représente un atome d'hydrogène ; un radical hydroxyle ou un radical alkyle en C₁-C₈, linéaire ou ramifié, saturé ou insaturé,
- p est 0 ou 1,
- les radicaux R, identiques ou différents désignent un groupe alkyle, linéaire ou ramifié, saturé ou insaturé en C₁-C₂₀ ; un groupe phényle ; un groupe trifluoro-3,3,3 propyle ou un groupe triméthylsilyloxy ; au moins 80% en nombre des radicaux R étant méthyle,
- B, identiques ou différents, sont choisis parmi les radicaux R et le radical A,
- r est un nombre variant de 0 à 50 ,
- s est un nombre variant de 0 à 20 et si s est 0, au moins un des deux symboles B est A,
- u est un nombre variant 1 à 6 inclus,
- t est un nombre variant de 0 à 10 ;
- t + u est supérieur ou égal à 3 .
- le radical R₂ désigne hydrogène ; un radical alkyle en C₁-C₂₀, linéaire ou ramifié , saturé ou insaturé ,
- le radical R₃ désigne un radical alkyle en C₁-C₂₀, linéaire ou ramifié, saturé ou insaturé ; un aryle en C₆-C₁₂,
- les radicaux R₄ et R₅, identiques ou différents, représentent hydrogène ; un radical alkyle en C₁-C₂₀, linéaire ou ramifié , saturé ou insaturé,
- le radical **R₆ est** un radical alkyle en C₁-C₂₀, linéaire ou ramifié , saturé ou insaturé ; un aryle en C₆-C₁₂ ;
sous réserve que lorsque 2 groupements (C=O)-X-L- sont présents, L n'est relié qu'à un seul groupement A ou B des formules (1) ou (2) : c'est à dire que s = 1 avec B étant différent de A ou s = 0 et un seul B est A pour la formule (1) et u = 1 pour la formule (2).

Bien que dans la formule (3) ci-dessus, seuls soient représentés les isomères dans lesquels le substituant Z est en position cis par rapport au substituant diaryle, cette formule doit être comprise comme englobant également l'isomère trans correspondant.

Dans les formules (1) et (2) ci-dessus, les radicaux alkyles peuvent être linéaires ou ramifiés, saturés ou insaturés et choisis notamment au sein des radicaux méthyle, éthyle, n-propyle, isopropyle, n-butyle, isobutyle, ter.-butyle, n-amyle, isoamyle, néopentyle, n-hexyle, n-heptyle, n-octyle, éthyl-2 hexyle et ter.-octyle. Le radical alkyle particulièrement préféré est le radical méthyle.

Dans la formule (3) ci-dessus, les radicaux alcoxy peuvent être linéaires ou ramifiés et choisis notamment au sein des radicaux méthoxy, éthoxy, n-propoxy, isopropoxy, n-butoxy et isobutoxy. Le radical alcoxy particulièrement préféré est le radical méthoxy.

Dans la formule (1) ci-dessus, les radicaux aryle sont de préférence phényle.

Les diorganosiloxanes linéaires ou cycliques de formule (1) ou (2) rentrant dans le cadre de la présente invention, sont des oligomères ou polymères statistiques présentant de préférence au moins l'une, et encore plus préférentiellement l'ensemble, des caractéristiques suivantes :
- le radical Z désigne -(C=O)R₃, -CN ou -(C=O)-X-L- ,
- R₃ désigne un radical alkyle en C₁-C₄ et encore plus préférentiellement éthyle
- R est alkyle en C₁-C₄ et encore plus préférentiellement méthyle,
- B est alkyle en C₁-C₄ et encore plus préférentiellement méthyle (dans le cas où le composé est de formule (1))
- r varie de 0 à 10 inclusivement ; s varie de 0 à 6 inclusivement (dans le cas où le composé est de formule (1))
- t+u varie de 3 à 5 (dans le cas où le composé est de formule (2))
- n est 0,
- W désigne CH₂,
- p est 1
- Y désigne H ou CH₃.

Parmi les diorganosiloxanes de formule (1) préférés, on peut citer les composés A, B, C et D de formules suivantes :

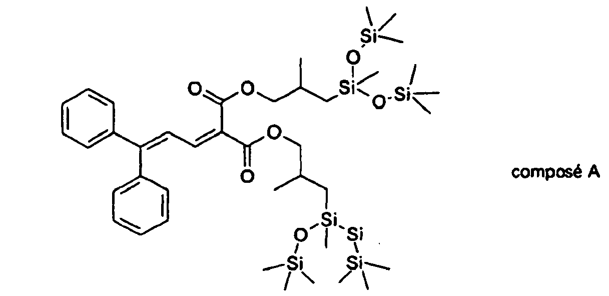

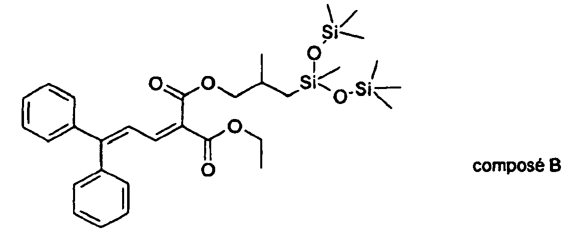

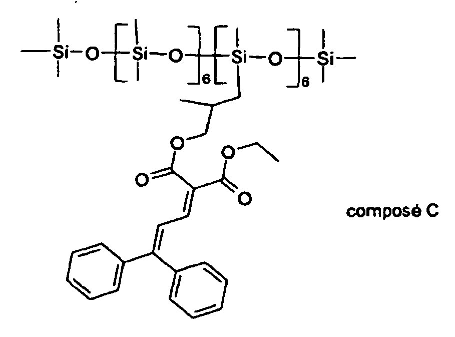

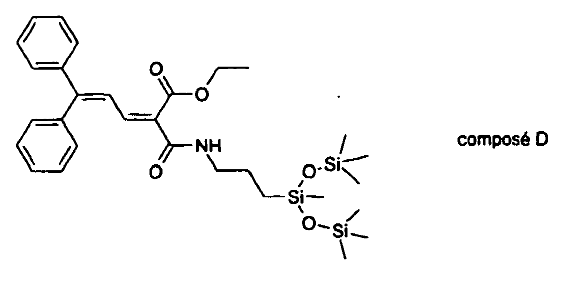

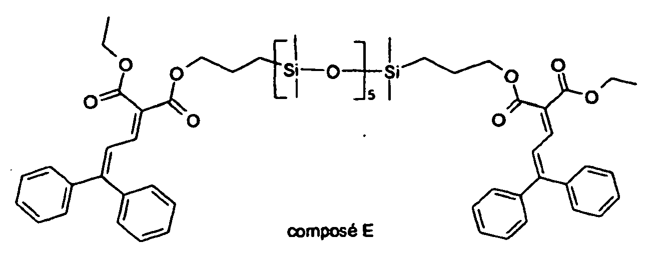

Une famille de composés diorganosiloxaniques à fonction 4,4-diarylbutadiène de formule (1) ou (2) particulièrement préférés est constituée par ceux pour lesquels Z désigne -CN. Ces composés particuliers sont efficaces dans la gamme des rayons UVA longs (340-400nm).

Parmi ces composés, on utilisera de préférence ceux présentant au moins l'une des caractéristiques suivantes et encore plus préférentiellement l'ensemble des caractéristiques suivantes :
- R est alkyle en C₁-C₄ et encore plus préférentiellement méthyle,
- B est alkyle en C₁-C₄ et encore plus préférentiellement méthyle (dans le cas où le composé est de formule (1 ))
- r varie de 0 à 10 inclusivement ; s varie de 0 à 6 inclusivement (dans le cas où le composé est de formule (1 ))
- t+u varie de 3 à 5 (dans le cas où le composé est de formule (2))
- n est 0,
- W désigne CH₂,
- p est 1
- Y désigne H ou CH₃.

Parmi ces composés de formule (1), on utilisera encore plus particulièrement les composés F, G, H, I, J suivants :

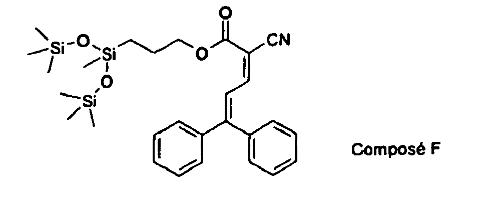

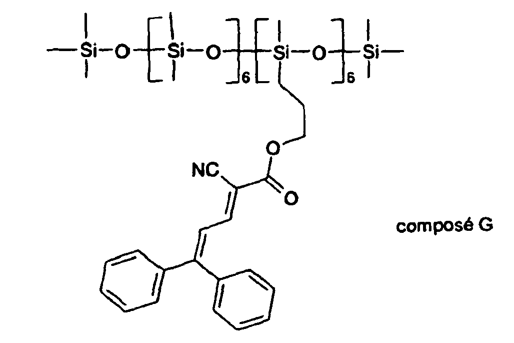

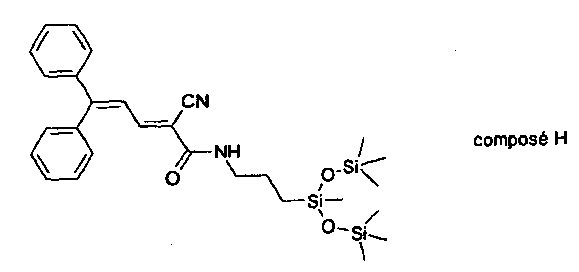

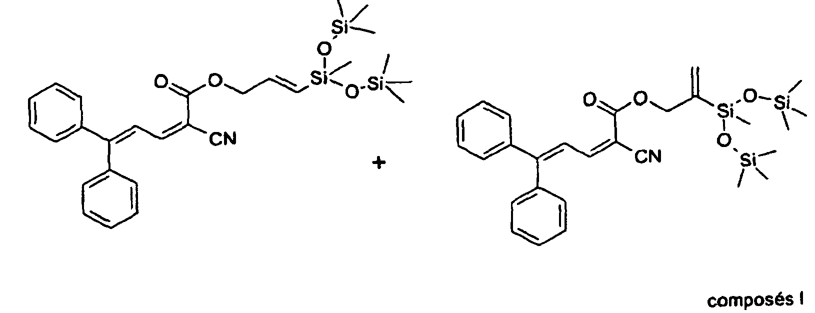

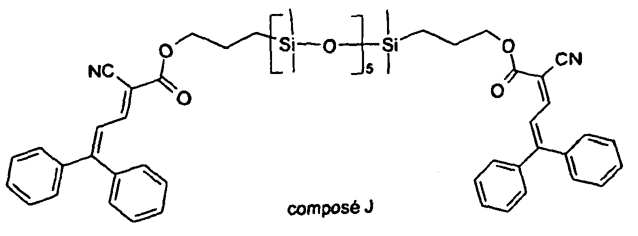

Pour préparer les dérivés de formule (1) ou (2) , on peut procéder classiquement en mettant en oeuvre une réaction d'hydrosilylation à partir du dérivé siloxanique correspondant dans lequel, par exemple, tous les radicaux A sont des atomes d'hydrogène. Ce dérivé est dénommé par la suite dérivé à SiH et la voie de synthèse correspondante est dénommée (Voie 1).

Les groupes SiH peuvent être présents dans la chaîne et/ou aux extrémités de la chaîne. Ces dérivés à SiH sont des produits bien connus dans l'industrie des silicones et sont généralement disponibles dans le commerce. Ils sont par exemple décrits dans les brevets américains US-A-3220972, US-A-3697473 et US-A-4340709.

Les dérivés à SiH correspondant respectivement aux composés de formules (1) et (2) peuvent être donc représentés par les formules (4) et (5) suivantes : 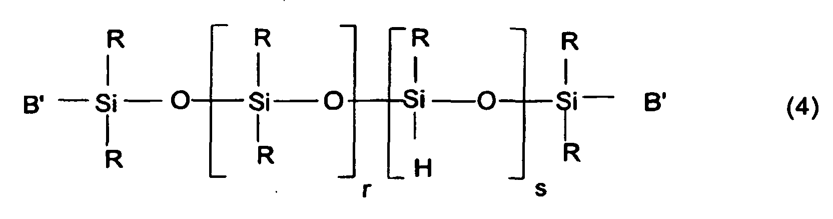 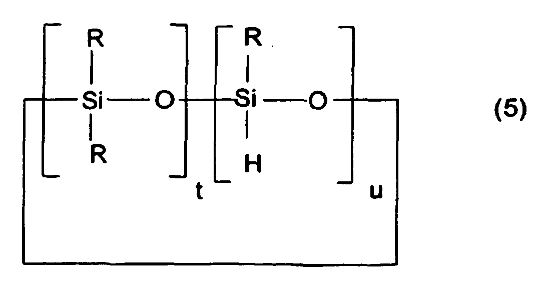 dans lesquelles :
- R, r, s, t et u ont la signification donnée ci-dessus pour les formules (1) et (2),
- B', identiques ou différents, sont choisis parmi les radicaux R et un atome d'hydrogène.

Afin de préparer les composés siloxaniques de l'invention de formules (1) ou (2) ci-dessus, on procède de la manière suivante : (Schéma 1) : sur le dérivé à SiH de formule (4) ou (5) correspondant, on effectue une réaction d'hydrosilylation en présence d'une quantité catalytiquement efficace d'un catalyseur au platine sur un dérivé organique de 4,4-diarylbutadiène de formule (6) suivante : 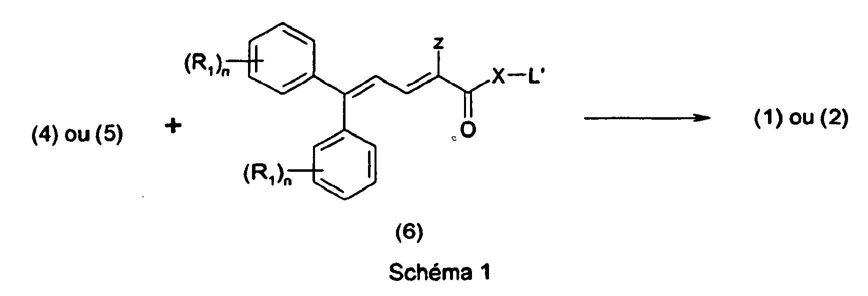 dans laquelle R₁ , Z, X et n ont la même signification qu'à la formule (3) ci-dessus et L' répond à l'une des deux formules (b) et (b') suivantes : 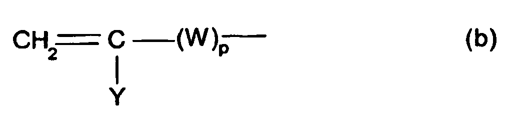 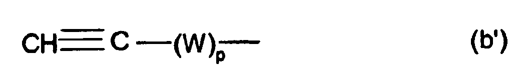 dans lesquelles Y, W et p ont les mêmes significations qu'aux formules (a), (a') et (a") ci-dessus.

La réaction d'hydrosilylation s'effectue donc selon l'une des deux réactions suivantes : 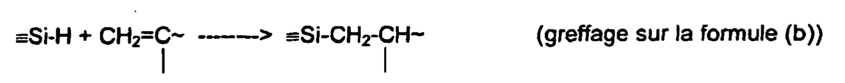 ou

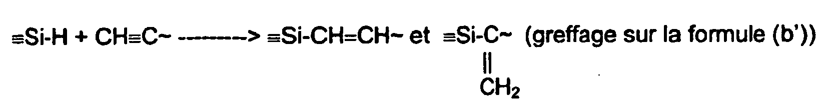

Les dérivés de formule (6) sont obtenus par condensation de Knoevenagel d'un dérivé de β-phényl cinnamaidéhyde sur un dérivé de formule (7) comme décrit dans le brevet EP 916335 : 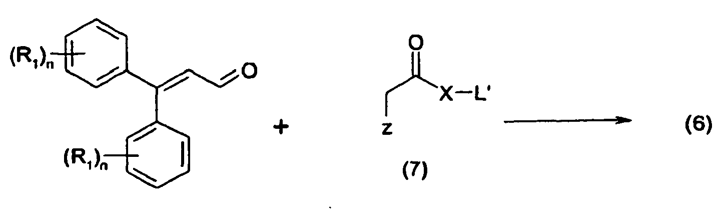 dans laquelle les radicaux R₁, Z, X, L' et n ont la même signification qu'aux formules (3) et (6) précédentes.

Les dérivés de formule (7) sont obtenus par alkylation des dérivés acides correspondants par des halogénures d'alcènes ou d'alcynes correspondants.

Comme dérivés de type Z-CH₂-X-L', on peut citer le malonate de diallyle (RN 1797-75-7), cyanoacetate d'allyle (RN 13361-32-5) ou l'acetoacetate de 2-propènyle (RN 1118-84-9) qui sont des produits commerciaux.

Dans le cas des dérivés amides de diorganosiloxanes (X représente NR₃) ,une autre voie de synthèse (Voie 2) d'obtention des composés de formules (1) ou (2) consiste à faire réagir un dérivé de β-phényl cinnamaldéhyde sur un aminodiorganosiloxane de formule (8) suivante : 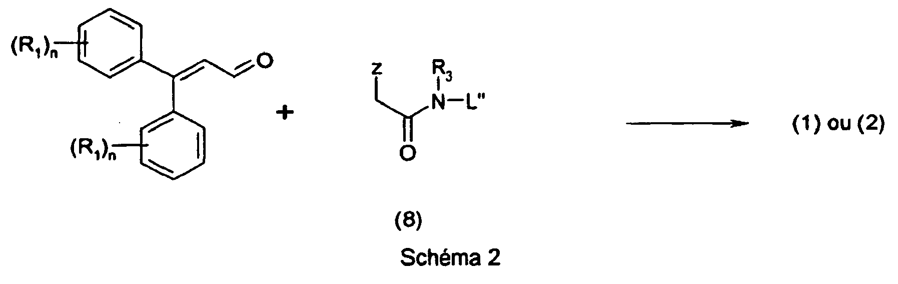 dans lequel les radicaux R₁, Z, R₃ et n ont la même signification qu'aux formules précédentes et L" répond à la formule (c) suivante : 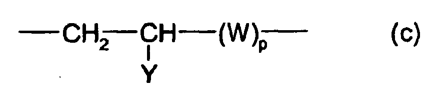 dans laquelle Y, W et p ont les mêmes significations qu'à la formule (a) ci-dessus.

Ces dérivés (8) pouvant être obtenus par condensation d'un chlorure d'acide sur les amino diorganosiloxanes de formule (9) suivante : 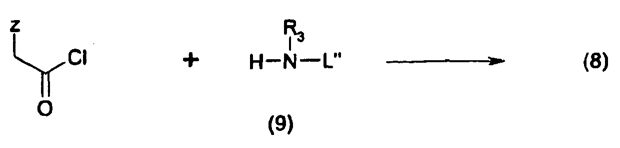 dans laquelle Z, R₃ et L" ont les mêmes significations qu'aux formules ci-dessus.

Comme dérivés de type Z-CH₂-COCl, on peut citer le chlorure de l'acide cyanoacétique ou le chlorure de l'acide monoéthyl malonique qui sont des produits commerciaux.

La préparation des aminosiloxanes de formule (9) est par exemple décrite dans GB 2 185 984. Comme aminosiloxanes convenant particulièrement bien à la préparation des composés selon l'invention, on peut citer l'aminopropyl heptaméthyl trisiloxane, l'aminoisobutyl heptaméthyl trisiloxane, ou encore les triméthylsilylamo-diméthicone telles que : le produit vendu sous la dénomination commerciale « X2-8260 » par la société DOW CORNING, d'indice d'amine 2,8 meq/gramme ; le produit vendu sous la dénomination commerciale « SLM 55051/3 » par la société WACKER, d'indice d'amine 0,47 meq/gramme ; les PDMS diméthylalkyle en C₁₂, telles que le produit vendu sous la dénomination commerciale « SLM 23046/1 » par la société WACKER, d'indice d'amine 1,2 meq/gramme ; les polyméthylalkyl (gras) arylalkylsiloxane α, ω triméthylées, telles que le produit vendu sous la dénomination commerciale « SLM 23056/2 » par la société WACKER, d'indice d'amine 1,3 meq/gramme ; les PDMS dont le radical NH₂ est en position α et ω sur un site alkyle telles que les produits vendus sous les dénominations commerciales «TEGOMER A-SI 2120 », d'indice d'amine 1,95 meq/gramme et « TEGOMER A-SI 2320 », d'indice d'amine 0,86 meq/gramme, par la société GOLDSCHMIDT.

La préparation des aminosiloxanes cycliques est par exemple décrite dans l'article de A. Kopylov, Zh.Obshch. Khim., 54(2), 367-71 (1984).

Les composés de formule (1) sont généralement présents dans la composition de l'invention dans des proportions comprises entre 0,01 % et 20 % en poids, de préférence entre 0,1 % et 10 % en poids, par rapport au poids total de la composition.

Les compositions conformes à l'invention peuvent comporter en plus d'autres filtres UV organiques ou inorganiques complémentaires actifs dans l'UVA et/ou l'UVB hydrosolubles, liposolubles ou bien insolubles dans les solvants cosmétiques couramment utilisés.

Les filtres organiques complémentaires sont notamment choisis parmi les anthranilates ; les dérivés cinnamiques ; les dérivés de dibenzoylméthane ; les dérivés salicyliques, les dérivés du camphre ; les dérivés de triazine tels que ceux décrits dans les demandes de brevet US 4367390, EP863145, EP517104, EP570838, EP796851, EP775698, EP878469, EP933376, EP507691, EP507692, EP790243, EP944624 ; les dérivés de la benzophénone ; les dérivés de β,β-diphénylacrylate; les dérivés de benzotriazole ; les dérivés de benzalmalonate ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans s les demandes US5,237,071, US 5,166,355, GB2303549, DE 197 26 184 et EP893119 ; les dérivés de benzoxazole tels que décrits dans les demandes de brevet EP0832642, EP1027883, EP1300137 et DE10162844 ; les polymères filtres et silicones filtres autres que ceux de formule (1) ou (2) tels que ceux décrits notamment dans la demande WO-93/04665 ; les dimères dérivés d'α-alkylstyrène tels que ceux décrits dans la demande de brevet DE19855649 ; les 4,4-diarylbutadiènes non siliciés tels que décrits dans les demandes EP0967200, DE19746654, DE19755649, EP-A-1008586, EP1133980 et EP133981 et leurs mélanges.

Comme exemples de filtres organiques complémentaires, on peut citer ceux désignés ci-dessous sous leur nom INCI :

### Dérivés de l'acide para-aminobenzoique :

PABA,
Ethyl PABA,
Ethyl Dihydroxypropyl PABA,
Ethylhexyl Diméthyl PABA vendu notamment sous le nom « ESCALOL 507 » par ISP, Glyceryl PABA,
PEG-25 PABA vendu sous le nom « UVINUL P25 » par BASF,

### Dérivés salicyliques :

Homosalate vendu sous le nom « Eusolex HMS » par Rona/EM Industries,
Ethylhexyl Salicylate vendu sous le nom « NEO HELIOPAN OS » par Haarmann et REIMER,
Dipropyleneglycol Salicylate vendu sous le nom « DIPSAL » par SCHER,
TEA Salicylate, vendu sous le nom « NEO HELIOPAN TS » par Haarmann et REIMER,

### Dérivés du dibenzoylméthane :

Butyl Methoxydibenzoylmethane vendu notamment sous le nom commercial « PARSOL 1789 » par HOFFMANN LA ROCHE,
Isopropyl Dibenzoylmethane,

### Dérivés cinnamiques :

Ethylhexyl Methoxycinnamate vendu notamment sous le nom commercial « PARSOL MCX » par HOFFMANN LA ROCHE,
Isopropyl Methoxy cinnamate,
Isoamyl Methoxy cinnamate vendu sous le nom commercial « NEO HELIOPAN E 1000 » par HAARMANN et REIMER,
Cinoxate,
DEA Methoxycinnamate,
- Diisopropyl Methylcinnamate,
Glyceryl Ethylhexanoate Dimethoxycinnamate

### Dérivés de β,β'-diphénylacrylate :

Octocrylene vendu notamment sous le nom commercial « UVINUL N539 » par BASF,
Etocrylene, vendu notamment sous le nom commercial « UVINUL N35 » par BASF,

### Dérivés de la benzophénone :

Benzophenone-1 vendu sous le nom commercial « UVINUL 400 » par BASF,
Benzophenone-2 vendu sous le nom commercial « UVINUL D50 » par BASF
Benzophenone-3 ou Oxybenzone, vendu sous le nom commercial « UVINUL M40 » par BASF,
Benzophenone-4 vendu sous le nom commercial « UVINUL MS40 » par BASF,
Benzophenone-5
Benzophenone-6 vendu sous le nom commercial « Helisorb 11 » par Norquay
Benzophenone-8 vendu sous le nom commercial « Spectra-Sorb UV-24 » par American Cyanamid
Benzophenone-9 vendu sous le nom commercial« UVINUL DS-49» par BASF,
Benzophenone-12
2-(4-diethylamino-2-hydroxybenzoyl)-benzoate de n-hexyle.

### Dérivés du benzylidène camphre :

3-Benzylidene camphor fabriqué sous le nom « MEXORYL SD» par CHIMEX,
4-Methylbenzylidene camphor vendu sous le nom « EUSOLEX 6300 » par MERCK ,
Benzylidene Camphor Sulfonic Acid fabriqué sous le nom « MEXORYL SL» par CHIMEX, Camphor Benzalkonium Methosulfate fabriqué sous le nom « MEXORYL SO » par CHIMEX,
Terephthalylidene Dicamphor Sulfonic Acid fabriqué sous le nom « MEXORYL SX » par CHIMEX,
Polyacrylamidomethyl Benzylidene Camphor fabriqué sous le nom « MEXORYL SW » par CHIMEX,

### Dérivés du phenyl benzimidazole :

Phenylbenzimidazole Sulfonic Acid vendu notamment sous le nom commercial « EUSOLEX 232 » par MERCK,
Disodium Phenyl Dibenzimidazole Tetra-sulfonate vendu sous le nom commercial commercial « NEO HELIOPAN AP » par Haarmann et REIMER,

### Dérivés de la triazine :

Anisotriazine vendu sous le nom commercial «TINOSORB S » par CIBA GEIGY,
Ethylhexyl triazone vendu notamment sous le nom commercial «UVINUL T150 » par BASF,
2,4,6-tris-(4'-amino benzalmalonate de diisobutyle)-s- triazine,
Diethylhexyl Butamido Triazone vendu sous le nom commercial « UVASORB HEB » par SIGMA 3V,

### Dérivés du phenyl benzotriazole :

Drometrizole Trisiloxane vendu sous le nom « Silatrizole » par RHODIA CHIMIE ,
Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,

### Dérivés anthraniliques :

Menthyl anthranilate vendu sous le nom commercial commercial « NEO HELIOPAN MA » par Haarmann et REIMER,

### Dérivés d'imidazolines :

Ethylhexyl Dimethoxybenzylidene Dioxoimidazoline Propionate,

### Dérivés du benzalmalonate :

Polyorganosiloxane à fonction benzalmalonate comme le produit Polysilicone-15 vendu sous la dénomination commerciale « PARSOL SLX » par HOFFMANN LA ROCHE

### Dérivés de 4,4-diarylbutadiène non siliciés :

1,1-dicarboxy (2,2'-diméthyl-propyl)-4,4-diphénylbutadiène

### Dérivés de benzoxazole :

2,4-bis-[5-1(diméthylpropyl)benzoxazol-2-yl-(4-phenyl)-imino]-6-(2-ethylhexyl)-imino-1,3,5-triazine vendu sous le nom Uvasorb K2A par Sigma 3V ;
et leurs mélanges.

Les filtres UV organiques complémentaires préférentiels sont choisis parmi
Ethylhexyl Salicylate,
Ethylhexyl Methoxycinnamate
Butyl Methoxydibenzoylmethane
Octocrylene,
Phenylbenzimidazole Sulfonic Acid,
Benzophenone-3,
Benzophenone-4,
Benzophenone-5,
2-(4-diethylamino-2-hydroxybenzoyl)-benzoate de n-hexyle.
4-Methylbenzylidene camphor,
Terephthalylidene Dicamphor Sulfonic Acid,
Disodium Phenyl Dibenzimidazole Tetra-sulfonate,
la 2,4,6-tris-(4'-amino benzalmalonate de diisobutyle)-s-triazine
Anisotriazine,
Ethylhexyl triazone,
Diethylhexyl Butamido Triazone,
Methylène bis-Benzotriazolyl Tetramethylbutylphénol
Drometrizole Trisiloxane
Polysilicone-15
1,1-dicarboxy (2,2'-diméthyl-propyl)-4,4-diphénylbutadiène
2,4-bis-[5-1 (diméthylpropyl)benzoxazol-2-yl-(4-phenyl)-imino]-6-(2-ethylhexyl)-imino-1,3,5-triazine
et leurs mélanges.

Les filtres complémentaires inorganiques sont choisis parmi des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium ou leurs mélanges qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP518772 et EP518773.

Les filtres UV complémentaires conformes à l'invention sont généralement présents dans les compositions selon l'invention dans des proportions allant de 0,01 à 20% en poids par rapport au poids total de la composition, et de préférence allant de 0,1 à 10% en poids par rapport au poids total de la composition.

Les compositions cosmétiques selon l'invention peuvent contenir en outre des agents de bronzage et/ou de brunissage artificiel de la peau (agents autobronzants) tels que la dihydroxyacétone (DHA). Ils sont présents de préférence dans des quantités allant de 0,1 à 10% et plus préférentiellement de 0,2 à 8% en poids par rapport au poids total de la composition.

Les compositions conformes à la présente invention peuvent comprendre en outre des adjuvants cosmétiques classiques notamment choisis parmi les corps gras, les solvants organiques, les épaississants ioniques ou non ioniques, les adoucissants, les humectants, les antioxydants, les hydratants, les desquamants, les agents anti-radicalaires, les agents antipollution, les antibactériens, les agents anti-inflammatoires, les dépigmentants, les pro-pigmentants, les opacifiants, les stabilisants, les émollients, les silicones, les agents anti-mousse, les agents répulsifs contre les insectes, les parfums, les conservateurs, les tensioactifs anioniques, cationiques, non-ioniques, zwitterioniques ou amphotères, les antagonistes de substance P, les antagonistes de substance CGRP, les charges, les pigments, les polymères, les propulseurs, les agents alcalinisants ou acidifiants ou tout autre ingrédient habituellement utilisé dans le domaine cosmétique et/ou dermatologique.

Les corps gras peuvent être constitués par une huile ou une cire ou leurs mélanges. Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (paraffine); végétales (huile d'amande douce, de macadamia, de pépin de cassis, de jojoba) ; synthétiques comme le perhydrosqualène, les alcools, les acides ou les esters gras (comme le benzoate d'alcools en C₁₂-C₁₅ vendu sous la dénomination commerciale « Finsolv TN » par la société WITCO, le palmitate d'octyle, le lanolate d'isopropyle, les triglycérides dont ceux des acides caprique/caprylique), les esters et éthers gras oxyéthylénés ou oxypropylénés; les huiles siliconées (cyclométhicone, polydiméthysiloxanes ou PDMS) ou fluorées, les polyalkylènes.

Comme composés cireux, on peut citer la paraffine, la cire de carnauba, la cire d'abeille, l'huile de ricin hydrogénée.

Parmi les solvants organiques, on peut citer les alcools et polyols inférieurs. Ces derniers peuvent être choisis parmi les glycols et les éthers de glycol comme l'éthylène glycol, le propylène glycol, le butylène glycol, le dipropylène glycol ou le diéthylène glycol.

Les épaississants peuvent être choisis notamment parmi les polymères acryliques réticulés comme les Carbomer, les polymères réticulés acrylates/C₁₀-C₃₀alkylacrylates du type Pemulen ou le polyacrylate-3 vendu sous le nom VISCOPHOBE DB 1000 par Amerchol ; les polyacrylamides tels que l'émulsion polyacrylamide, C₁₃-C₁₄ isoparraffine et laureth-7 vendue sous le nom SEPIGEL 305 par SEPPIC, les homopolymères ou copolymères d'AMPS tel l'HOSTACERIN AMPS vendu par CLARIANT, les gommes de guar et celluloses modifiées ou non telles que la gomme de guar hydroxypropylée, la méthylhydroxyéthylcellulose et l'hydroxypropylméthyl cellulose, la gomme de xanthane, les silices nanométriques de type Aerosil.

Bien entendu, l'homme de l'art veillera à choisir le ou les éventuels composés complémentaires cités ci-dessus et/ou leurs quantités de manière telle que les propriétés avantageuses attachées intrinsèquement aux composés conformes à l'invention ne soient pas, ou substantiellement pas, altérées par la ou les adjonctions envisagées.

Les compositions selon l'invention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait, ou sous la forme d'un gel ou d'un gel crème, sous la forme d'une lotion, d'une huile, de poudre, de bâtonnet solide et éventuellement être conditionnée en aérosol et se présenter sous forme de mousse ou de spray.

De préférence, les compositions selon l'invention se présentent sous la forme d'une émulsion huile-dans-eau ou eau-dans huile.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

Lorsque la composition cosmétique selon l'invention est utilisée pour le soin de l'épiderme humain, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, d'huile solaire, de bâtonnet solide, de poudre, de mousse aérosol ou de spray.

Lorsque la composition cosmétique selon l'invention est utilisée pour le soin des cheveux, elle peut se présenter sous forme de shampooing, de lotion, de gel, d'émulsion, de dispersion vésiculaire non ionique et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des ongles, des lèvres, des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

Un autre objet de l'invention est l'utilisation d'un composé de formule (1) tel que définie ci-dessus dans une composition cosmétique comme agent de contrôle de la variation de la couleur de la peau due aux rayonnements UV-A.

Un autre objet de l'invention est l'utilisation d'un composé de formule (1) tel que définie précédemment comme agent photostabilisant de polymères synthétiques tels que les matières plastiques ou de verres en particulier des verres de lunettes ou des lentilles de contact.

Des exemples concrets, mais nullement limitatifs, illustrant l'invention, vont maintenant être donnés.

### EXEMPLES DE PREPARATION

### EXEMPLE 1 : Préparation du diester mono ethyle mono 3-[1,3,3,3-tetraméthyl-1-[(triméthylsilyl)oxy]disiloxanyl]-2-méthyl-propyle de l'acide 2-(3,3-diphénylprop-2-énylène) malonique (selon la Voie 1):

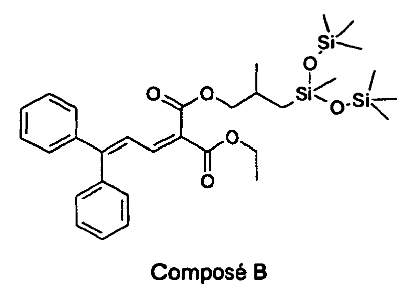

### Première étape : Préparation du diester mono éthyle mono méthallvle de l'acide malonique :

Dans un réacteur, on porte à 50°C pendant 4 heures un mélange du sel potassique de l'acide monoéthyle malonique (17 g, 0,1 mole) et du chlorure de méthallyle (9,8 ml, 0,1 mole) dans 150 ml de DMF. Les sels minéraux sont filtrés sur verre fritté et le DMF est évaporé sous pression réduite. Le mélange est repris dans 100 ml d'acétate d'éthyle. La phase organique est lavée 3 fois à l'eau. La phase organique est séchée sur sulfate de sodium et concentrée sous pression réduite. On obtient 16 g d'une huile jaune pâle qui est purifiée par distillation fractionnée. On récupère les fractions qui distillent à 58°C sous un vide de 0,1 hPa sous forme d'une huile incolore (8g, Rendement 40%) du diester mono éthyle mono méthallyle de l'acide malonique utilisé tel quel dans l'étape suivante.

### Deuxième étape : Préparation du diester mono éthyle mono méthallyle de l'acide 2-(3,3-diphénylprop-2-énylène) malonique :

Dans un réacteur on solubilise le β-phényl cinnamaldéhyde (2 g, 0,0096 mole) dans 50 ml d'isopropanol. On y ajoute 0,24 ml de pipéridine et le dérivé de la première étape (1,97 g, 0,011 mole). On chauffe le mélange réactionnel à 60°C pendant 2 heures et 30 minutes. L'isopropanol est éliminé sous pression réduite. On obtient après cristallisation dans l'heptane le dérivé diester mono éthyle mono méthallyle de l'acide 2-(3,3-diphénylprop-2-énylène) malonique (2,05 g, Rendement 57%) sous forme d'une poudre jaune pâle :
- ¹H RMN (400MHz) ; présence des 2 isomères E,Z dans le rapport 4 :1.
- Pf 95°C

### Troisième étape : Préparation du dérivé de l'exemple 1 :

On chauffe à 80°C une solution du produit précédent (1 g, 0,0026 mole) et de catalyseur (complexe à 3-3,5% poids de Pt dans du cyclovinylméthylsiloxane de Hüls Petrarch PC085 : 40 µl) dans 30 ml de toluène. On y ajoute goutte à goutte en 20 minutes 0,65 g (0,029 mole) d'heptaméthyltrisiloxane. Le mélange est chauffé à 80°C pendant 20 heures. On évapore le solvant sous vide. L'huile obtenue est purifiée par chromatographie sur colonne de Silice (éluant : CH₂Cl₂/Heptane). On récupère (0,5 g, Rendement 31%) les fractions propres du dérivé de l'exemple 1 sous forme d'une huile jaune pâle :
- ¹H RMN (400MHz) ; présence des 2 isomères E,Z dans le rapport 4 :1.
- UV(Ethanol) λₘₐₓ = 333 nm εₘₐₓ = 39 530 E1% = 660.

### EXEMPLE 2 : Préparation du dérivé statistique de formule (1) : s = 6, r = 6, B = CH₃, R = CH₃, n = 0, Z = -COOEt, X = O, L de formule (a) : Y = CH₃, p = 1, W = CH₂ (selon la Voie 1) :

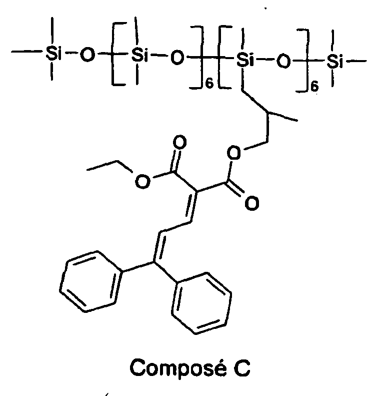

On chauffe à 90°C une solution du produit précédent (1 g, 0,0026 mole) et de catalyseur (complexe à 3-3,5% poids de Pt dans du cyclovinylméthylsiloxane de Hüls Petrarch PC085 : 40 µl) dans 30 ml de toluène. On y ajoute 0,42 g (2,5 meq SiH) du copolymère statistique (50-55%) méthylhydro - (45-50%) diméthylsiloxane (PS122.5 de Petrarch). Le mélange est chauffé à 90°C jusqu'à disparition de la bande à 2180 cm-1 en Infrarouge (soit 10 heures). On évapore le solvant sous vide. L'huile obtenue est lavée 2 fois à l'éthanol à 80%. L'huile jaune pâle obtenue est reprise dans le dichlorométhane, séchée sur sulfate de sodium et est passée sur lit de Silice. Après évaporation du solvant, on obtient (0,8 g, Rendement 56%) des fractions propres du dérivé de l'exemple 2 sous forme d'une huile jaune pâle :
- UV (Ethanol) λₘₐₓ = 333 nm, E1% = 590.

### EXEMPLE 3 : Préparation de l'ester 3-[1,3,3,3-tetraméthyl-1-[(triméthylsilyl)oxy]disiloxanyl]propyle de l'acide 2-cyano-5,5-diphénylpenta-2,4-diènoïque (selon la Voie 1) :

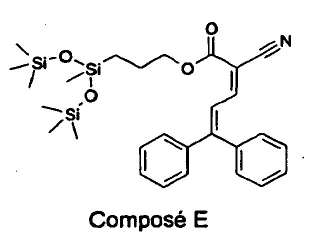

### Première étape : Préparation de l'ester allylipue de l'acide 2-cyano-5,5-diphénylpenta-2,4-diène pentanoïque :

Dans un réacteur on solubilise le β-phényl cinnamaldéhyde (4 g, 0,0192 mole) dans 100 ml d'isopropanol. On y ajoute 0,5 ml de pipéridine et le cyanoacétate d'allyle (2,64 g, 0,021 mole). On chauffe le mélange réactionnel à 60°C pendant 2 heures et 30 minutes. La solution est concentrée sous pression réduite. L'excès de malonate est éliminé par distillation sous un vide de 0,02 hPa. Après purification par chromatographie sur colonne de silice (éluant : Heptane/AcOEt 95 :5), on obtient les fractions propres de dérivé de l'ester allylique de l'acide 2-cyano-5,5-diphénylpenta-2,4-diène pentanoïque (2,34 g, Rendement 77%) sous forme d'une poudre jaune pâle et utilisée telle quelle dans l'étape suivante.

### Deuxième étatpe : Préparation du dérivé de l'exemple 3 :

On chauffe à 80°C une solution du produit précédent (1 g, 0,00317 mole) et de catalyseur (complexe à 3-3,5% poids de Pt dans du cyclovinylméthylsiloxane de Hüls Petrarch PC085 : 40 µl) dans 30 ml de toluène. On y ajoute goutte à goutte en 20 minutes 0,776 g (0,0349 mole) d'heptaméthyltrisiloxane. Le mélange est chauffé à 80°C pendant 20 heures. On évapore le solvant sous vide. L'huile obtenue est purifiée par chromatographie sur colonne de Silice (éluant : CH₂Cl₂/Heptane). On récupère (0,93 g, Rendement 55%) les fractions propres du dérivé de l'exemple 3 sous forme d'une huile jaune pâle :
- UV (Ethanol) λmax = 358 nm, εₘₐₓ = 30 874 E1% = 574.

### EXEMPLE 4 : Préparation du dérivé statistique de formule (1) : s = 6, r = 6, B = CH₃, R = CH₃, n = 0, Z = -CN, X = O, L de formule (a) : Y = H, p = 1, W = CH₂ (selon la Voie 1) :

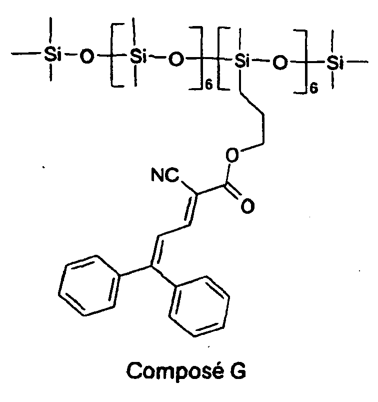

On chauffe à 90°C une solution du produit précédent (1 g, 0,00317 mole) et de catalyseur (complexe à 3-3,5% poids de Pt dans du cyclovinylméthylsiloxane de Hüls Petrarch PC085 : 40 µl) dans 30 ml de toluène. On y ajoute 0,52 g (3,1 meq SiH) du copolymère statistique (50-55%) méthylhydro - (45-50%) diméthylsiloxane (PS122.5 de Petrarch). Le mélange est chauffé à 90°C jusqu'à disparition de la bande à 2180 cm-1 en Infrarouge (soit 12 heures). On évapore le solvant sous vide. L'huile obtenue est lavée 2 fois à l'éthanol à 80%. L'huile jaune pâle obtenue est reprise dans le dichlorométhane, séchée sur sulfate de sodium et est passée sur lit de Silice. Après évaporation du solvant, on obtient (1,06 g, Rendement 62%) les fractions propres du dérivé de l'exemple 4 sous forme d'une huile jaune pâle :
- UV (Ethanol) λₘₐₓ = 359 nm, E1% = 660.

### EXEMPLE 5 : Préparation du N-[3-[1,3,3,3-tetraméthyl-1-[(triméthylsilyl)oxy]disiloxanyl]propyle]-2-cyano-5,5-diphénylpenta-2,4-diènenamide (selon la Voie 2) :

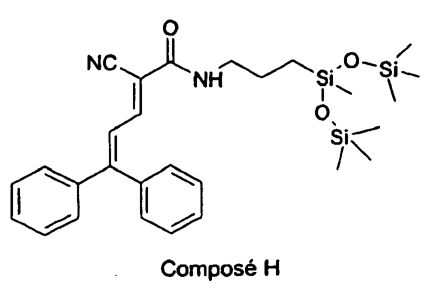

### Première étape : Préparation du cyano-N-[3-[1,3,3,3-tetraméthyl-1-[(triméthylsilyl)oxy]disiloxanyl]propyle]acetamide :

A un mélange de 3-[1,3,3,3-tetraméthyl-1-[(triméthylsilyl)oxy]disiloxanyl]-propylamine (13,93 g, 0,05 mole) et de triéthylamine (5 g, 0,05 mole) dans 40 ml de dichlorométhane, on ajoute goutte à goutte le chlorure de l'acide cyano acétique (5,2 g, 0,05 mole) en 20 minutes. La température monte jusqu'à 32°C. On laisse au reflux pendant 2 heures. Le mélange réactionnel est versé dans 150 ml d'eau et est extrait avec du dichlorométhane. Les phases organiques sont lavées à l'eau, séchées sur sulfate de sodium puis le solvant évaporé. Après purification par passage sur colonne de silice (éluant : Heptane/Acétate d'éthyle 70:30), on obtient 12,2 g (Rendement 70 %) du cyano-N-[3-[1,3,3,3-tetraméthyl-1-[(triméthylsilyl)oxy]disiloxanyl]propyle] acetamide d'une huile incolore utilisée telle quelle dans l'étape suivante.

### Deuxième étape : Préparation du dérivé de l'exemple 5 :

Dans un réacteur on solubilise le β-phényl cinnamaldéhyde (0,6 g, 2,88x10⁻³ mole) dans 30 ml d'isopropanol. On y ajoute 0,1 ml de pipéridine et le dérivé de l'étape précédente (1 g, 2,88x10⁻³ mole). On chauffe le mélange réactionnel à 50°C pendant 1 heure et 30 minutes. La solution est concentrée sous pression réduite. Après purification par chromatographie sur colonne de silice (éluant : Heptane/AcOEt 90 :10), on obtient les fractions propres du dérivé de l'exemple (0,95 g, Rendement 61%) sous forme d'une huile jaune pâle :
- UV (Ethanol) λₘₐₓ = 351 nm, εₘₐₓ = 27 650, E1% = 515.

### EXEMPLE 6 : Préparation du diester [2-méthyl-3-[1,3,3,3-tetraméthyl-1-[(triméthylsilyl)oxy]disiloxanyl]-2-méthyle-propyle de l'acide 2-(3,3-diphénylprop-2-énylène) malonique (selon la Voie 1) :

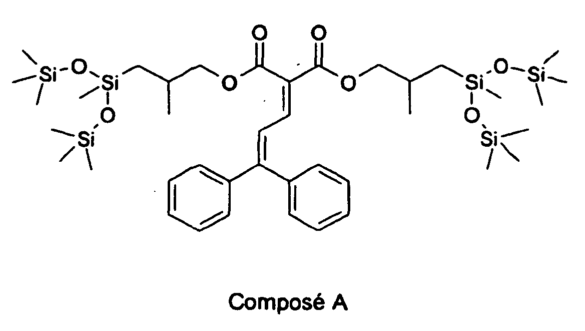

### Première étape: Préparation du malonate de 2-méthyl-3-[1,3,3,3-tetraméthyl-1-[(triméthylsilyl)oxy]disiloxanyl]propyle :

On chauffe à 80°C une solution de malonate de diméthallyle (10 g, 0,047 mole) et de catalyseur (complexe à 3-3.5% poids de Pt dans du cyclovinylméthylsiloxane de Hüls Petrarch PC085 : 200 µl) dans 30 ml de toluène. On y ajoute goutte à goutte en 20 minutes 23 g (0,104 mole) d'heptaméthyltrisiloxane. Le mélange est chauffé à 80°C pendant 16 heures. On évapore sous vide le solvant et l'excès d'heptaméthyltrisiloxane. On obtient ainsi le di-[2-méthyl-3-[1,3,3,3-tetraméthyl-1-[(triméthylsilyl)oxy]disiloxanyl]propyl]-malonate sous forme d'une huile laquelle est utilisée telle quelle pour l'étape suivante.

### Deuxième étape : Préparation du dérivé de l'exemple 6 :

On porte au reflux pendant 12 heures un mélange du dérivé précédent (1 g, 0,00234 mole) et de β-phényl cinnamaldéhyde (0,487 g, 0.00234 mole) dans 20 ml de toluène en présence de 0,1 ml de pipéridine et de 0,06 ml d'acide acétique. On concentre le mélange réactionnel et on effectue une chromatographie sur silice (éluant : Heptane/CH₂Cl₂ 50:50) pour obtenir 0,62 g du dérivé de l'exemple 6 (Rendement : 32%) sous forme d'une huile jaune pâle :
- UV (Ethanol) λₘₐₓ = 330 nm, εₘₐₓ = 28 650, E1% = 346.

### EXEMPLE 7 : Préparation de l'ester éthylique du 4,4-diphényl-1,3-diène N-[3-[1,3,3,3-tetraméthyl-1-[(triméthylsilyl)oxy]disiloxanyl]propyle] de l'acide 2-(4,4-diphénylprop-2éthylène) malonamide (selon la Voie 2) :

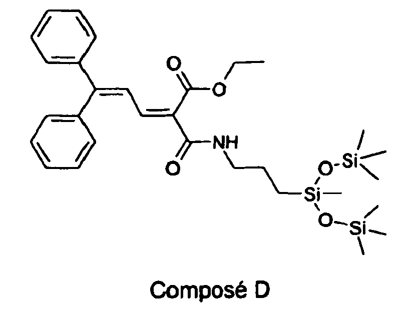

### Première étape : préparation de l'ester éthylique du N-[3-[1,3,3,3-tetraméthyl-1-[(triméthylsilyl)oxy]disiloxanyl]propyl]-malonamide

A un mélange de 3-[1,3,3,3-tetraméthyl-1-[(triméthylsilyl)oxy]disiloxanyl]-propylamine (6,99 g, 0,025 mole) et de triéthylamine (2,2 g, 0,0275 mole) dans 20 ml de dichlorométhane, on ajoute goutte à goutte à 10°C le chlorure de l'acide malonique monoéthyl ester (3,3 g, 0,025 mole) en 30 minutes. On laisse remonter le mélange réactionnel à température ambiante et on porte au reflux pendant 2 heures. Le mélange réactionnel est versé dans 40 ml d'eau et est extrait avec du dichlorométhane. Les phases organiques sont lavées à l'eau, séchées sur sulfate de sodium puis le solvant évaporé. Après purification par passage sur colonne de silice (éluant : CH₂Cl₂/Méthanol 95:5), on obtient 2,1 g d'une huile incolore utilisée telle quelle dans l'étape suivante.

### Deuxième étape : Préparation du dérivé de l'exemple 7 :

On porte à 50°C pendant 3 heures un mélange du dérivé précédent (1 g, 2,54x10⁻³ mole) et de β-phényl cinnamaldéhyde (0,53 g, 2,54x10⁻³ mole) dans 30 ml d'alcool isopropylique en présence de 0,1 ml de pipéridine. On concentre le mélange réactionnel et on effectue une chromatographie sur silice (éluant : Heptane/Acétate d'éthyle 80:20) pour obtenir 1,22 g (Rendement 82%) du dérivé de l'exemple 7 sous forme d'une huile jaune pâle :
- UV (Ethanol) λₘₐₓ = 335 nm, εₘₐₓ = 31 650, E1% = 542.

### EXEMPLES DE COMPOSITION

### Composition A

| **Phase** | **Constituants** | **Concentration** **(g%)** |
|---|---|---|
| A | Mélange monostéarate de glycéryle / Stéarate de polyéthylène glycol (100 OE) (ARLACEL 165 - Uniquema) | 1 |
| | Acides gras d'origine végétale (STEARINE TP 1200 - Stearinerie Dubois) | 1.5 |
| | Diméthicone (DOW CORNING 200 FLUID 350 CS - Dow Corning) | 0.5 |
| | Alcool cétylique (LANETTE 16 - Cognis) | 0.5 |
| | Mélange cétylstéarylglucoside/ Alcool cétylstéarylique (MONTANOV 68 - Seppic) | 2 |
| | Benzoate d'alcools en C₁₂-C₁₅ FINSOLV TN - Witco | 10 |
| | **Composé de l'exemple 1** | 5 |
| B | Glycérine (PRICERINE 9091 - Uniquema) | 5 |
| | Phosphate d'alcool hexadecylique, sel de potassium (AMPHISOL K - Roche Vitamins) | 1 |
| | EDTA | 0.1 |
| C | Gomme de xanthane (KELTROL T - CP KELCO) | 0.2 |
| | Copolymère réticulé Acrylates/C₁₀-C₃₀-alkylacrylate (PEMULEN TR-1 - Novéon) | 0.2 |
| | Isohexadécane (ISOHEXADECANE - BP) | 1 |
| | Triethanolamine | qs pH |
| | Conservateurs | qs |
| | Eau déminéralisée | qsp 100g |

### Protocole de fabrication au laboratoire:

On pèse la phase grasse (A) et on chauffe au bain marie à 70°C. On pèse la phase aqueuse (B) dans le bêcher final et on chauffe au bain marie à 70°C. Sous agitation Rotor/Stator type MORITZ, on disperse la phase grasse dans la phase aqueuse (environ 1000 tr/min.). On incorpore le mélange d'épaississants (C) et on laisse revenir à température ambiante sous agitation.Vers 30°C, on neutralise la formule et on conditionne.

### Composition B

| **Phase** | **Constituants** | **Concentration** **(g%)** |
|---|---|---|
| A | Mélange monostéarate de glycéryle / Stéarate de polyéthylène glycol (100 OE) (ARLACEL 165 - Uniquema) | 1 |
| | Acides gras d'origine végétale (STEARINE TP 1200 - Stearinerie Dubois) | 1.5 |
| | Diméthicone (DOW CORNING 200 FLUID 350 CS - Dow Corning) | 0.5 |
| | Alcool cétylique (LANETTE 16 - Cognis) | 0.5 |
| | Mélange cétylstéarylglucoside/ Alcool cétylstéarylique (MONTANOV 68 - Seppic) | 2 |
| | Benzoate d'alcools en C₁₂-C₁₅ FINSOLV TN - Witco | 10 |
| | **Composé de l'exemple 3** | 5 |
| B | Glycérine (PRICERINE 9091 - Uniquema) | 5 |
| | Phosphate d'alcool hexadecylique, sel de potassium (AMPHISOL K - Roche Vitamins) | 1 |
| | EDTA | 0.1 |
| C | Gomme de xanthane (KELTROL T - CP KELCO) | 0.2 |
| | Copolymère réticulé Acrylates/C₁₀-C₃₀-alkylacrylate (PEMULEN TR-1 - Novéon) | 0.2 |
| | Isohexadécane (ISOHEXADECANE - BP) | 1 |
| | Triethanolamine | qs pH |
| | Conservateurs | qs |
| | Eau déminéralisée | qsp 100g |

### Protocole de fabrication au laboratoire:

On pèse la phase grasse (A) et on chauffe au bain marie à 70°C. On pèse la phase aqueuse (B) dans le bêcher final et on chauffe au bain marie à 70°C. Sous agitation Rotor/Stator type MORITZ, on disperse la phase grasse dans la phase aqueuse (environ 1000 tr/min.). On incorpore le mélange d'épaississants (C) et on laisse revenir à température ambiante sous agitation. Vers 30°C, on neutralise la formule et on conditionne.

## Revendications

1. Composé diorganosiloxanique à fonction 4,4-diarylbutadiène, sous forme isolée ou de mélange correspondant à l'une des formules générales (1) ou (2) suivante : 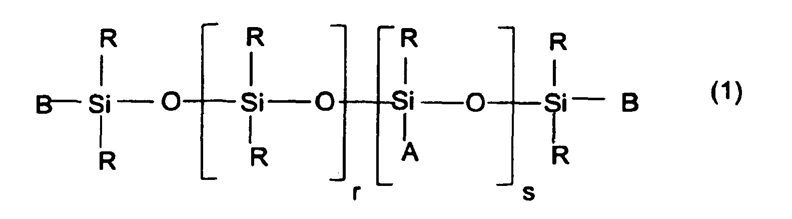 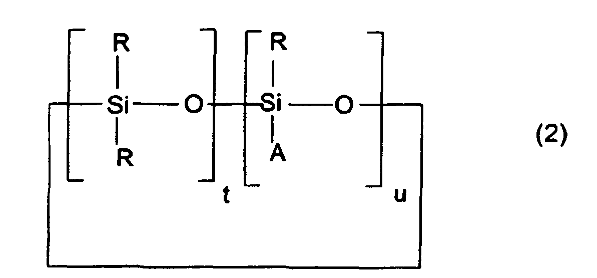 dans lesquelles :
- A désigne un radical de formule (3) suivante : 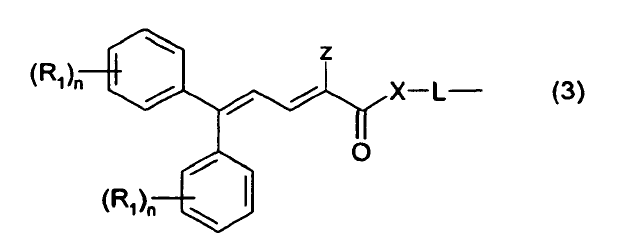
dans laquelle :
- les radicaux R₁ , égaux ou différents, désignent un radical hydroxyle ; un radical alkyle en C₁-C₁₀ , linéaire ou ramifié, saturé ou insaturé ; un radical alcoxy en C1-C10 linéaire ou ramifié, saturé ou insaturé, 2 groupes R₁ adjacents pouvant former ensemble un cycle alkylène dioxy en C₁-C₃ ;
- n est un entier allant de 0 à 2 ;
- le radical Z désigne hydrogène ; -(C=O)OR₂ , -(C=O)R₃, -(C=O)NR₄R₅ , -SO₂R₆ , -CN ou -(C=O)X-L- ,
- X désigne -O- ou -NR₄-,
- L est un radical divalent permettant l'accrochage du radical A sur la chaîne siliconée répondant à l'une des formules (a) , (a') ou (a") suivantes : 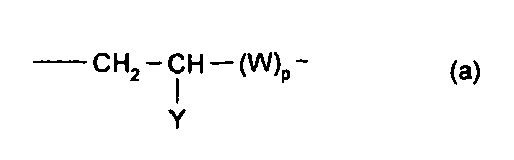
—CH=CH-(W)ₚ- (a') 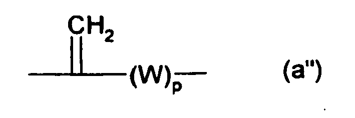
dans lesquelles :
- W est un radical alkylène en C₁-C₆ linéaire ou ramifié, saturé ou insaturé, éventuellement substitué par un radical hydroxyle ou un radical alkyle en C₁-C₈, linéaire ou ramifié, saturé ou insaturé,
- Y représente un atome d'hydrogène ; un radical hydroxyle ou un radical alkyle en C₁-C₈, linéaire ou ramifié, saturé ou insaturé,
- p est 0 ou 1,
- les radicaux R, identiques ou différents désignent un groupe alkyle, linéaire ou ramifié, saturé ou insaturé en C₁-C2₀ ; un groupe phényle ; un groupe trifluoro-3,3,3 propyle ou un groupe triméthylsilyloxy ; au moins 80% en nombre des radicaux R étant méthyle,
- B, identiques ou différents, sont choisis parmi les radicaux R et le radical A,
- r est un nombre variant de 0 à 50 ,
- s est un nombre variant de 0 à 20 et si s est 0, au moins un des deux symboles B est A,
- u est un nombre variant 1 à 6 inclus,
- t est un nombre variant de 0 à 10 ;
- t+ u est supérieur ou égal à 3 .
- le radical R₂ désigne hydrogène ; un radical alkyle en C1-C20, linéaire ou ramifié , saturé ou insaturé ,
- le radical R₃ désigne un radical alkyle en C1-C20, linéaire ou ramifié , saturé ou insaturé ; un aryle en C₆-C₁₂,
- les radicaux R₄ et R₅, identiques ou différents, représentent hydrogène ; un radical alkyle en C₁-C₂₀, linéaire ou ramifié, saturé ou insaturé,
- le radical **R₆ est** un radical alkyle en C₁-C₂₀, linéaire ou ramifié , saturé ou insaturé ; un aryle en C₆-C₁₂,
sous réserve que lorsque 2 groupements (C=O)-X-L- sont présents, L n'est relié qu'à un seul groupement A ou B des formules (1) ou (2) : c'est à dire que s = 1 avec B étant différent de A ou s = 0 et un seul B est A pour la formule (1) et u = 1 pour la formule (2).

2. Composé diorganosiloxanique selon la revendication 1, présentant au moins l'une, et encore plus préférentiellement l'ensemble, des caractéristiques suivantes :
- le radical Z désigne -(C=O)R₃, -CN ou -(C=O)-X-L- ,
- R₃ est alkyle en C₁-C₄ et encore plus préférentiellement éthyle,
- R est alkyle en C₁-C₄ et encore plus préférentiellement méthyle,
- B est alkyle en C₁-C₄ et encore plus préférentiellement méthyle (dans le cas où le composé est de formule (1))
- r varie de 0 à 10 inclusivement ; s varie de 0 à 6 inclusivement (dans le cas où le composé est de formule (1)),
- t+u varie de 3 à 5 (dans le cas où le composé est de formule (2)),
- n est 0,
- W désigne CH₂,
- p est 1
- Y désigne H ou CH₃.

3. Composé diorganosiloxanique à fonction 4,4-diarylbutadiène de formule (1) selon la revendication 1 ou 2 choisi dans le groupe constitué des composés A, B, C, D **et E** suivants

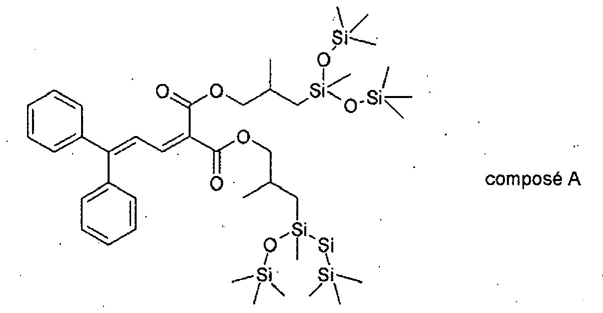

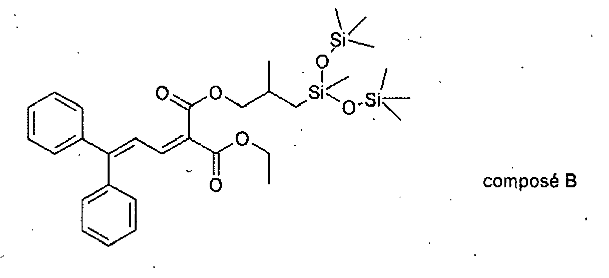

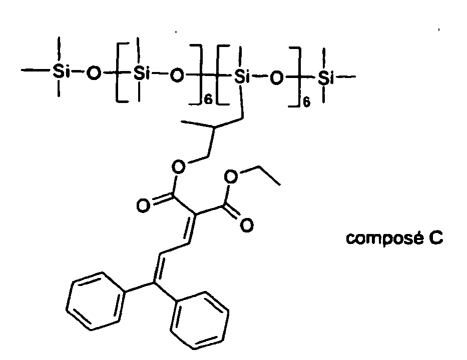

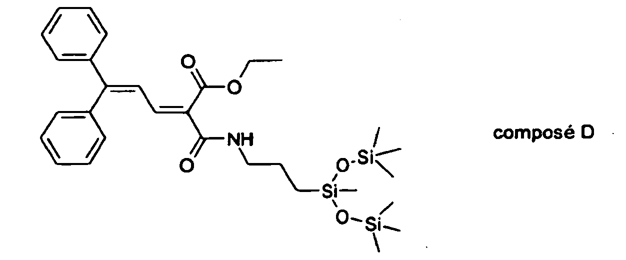

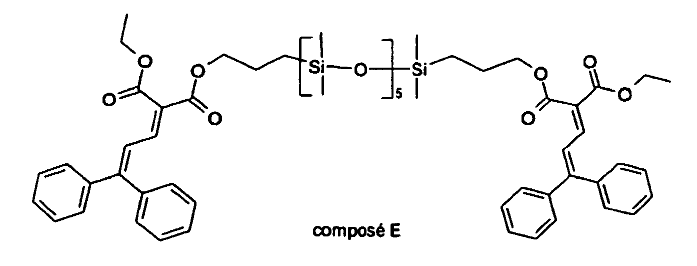

4. Composé diorganosiloxanique à fonction 4,4-diarylbutadiène de formule (1) ou (2) selon la revendication 1 ou 2, où Z désigne CN.

5. Composé diorganosiloxanique à fonction 4,4-diarylbutadiène de formule (1) ou (2) selon la revendication 4 présentant au moins l'une, et encore plus préférentiellement l'ensemble des caractéristiques suivantes :
- R est alkyle en C₁-C₄ et encore plus préférentiellement méthyle,
- B est alkyle en C₁-C₄ et encore plus préférentiellement méthyle (dans le cas où le composé est de formule (1))
- r varie de 0 à 10 inclusivement ; s varie de 0 à 6 inclusivement (dans le cas où le composé est de formule (1)),
- t+u varie de 3 à 5 (dans le cas où le composé est de formule (2)),
- n est 0,
- W désigne CH₂,
- p est 1
- Y désigne H ou CH₃.

6. Composé diorganosiloxanique à fonction 4,4-diarylbutadiène selon la revendication 1 ou 2 choisi dans le groupe constitué des composés F, G, H, I, J suivants :

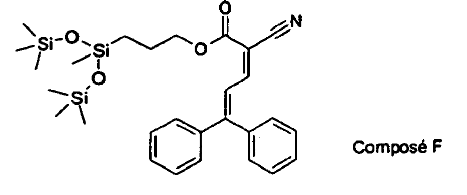

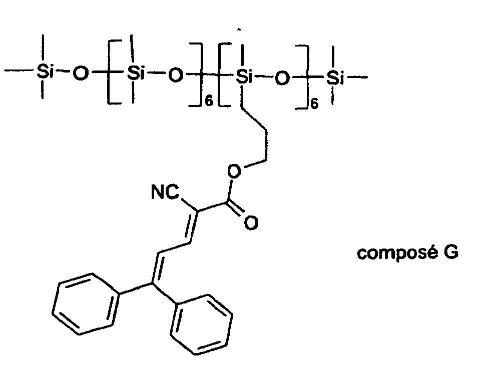

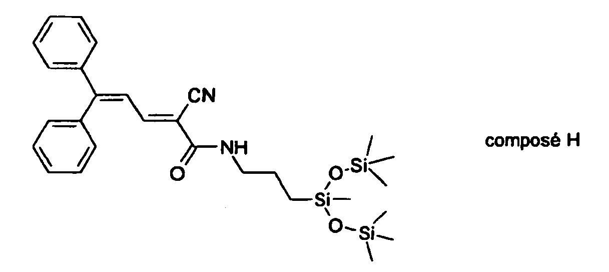

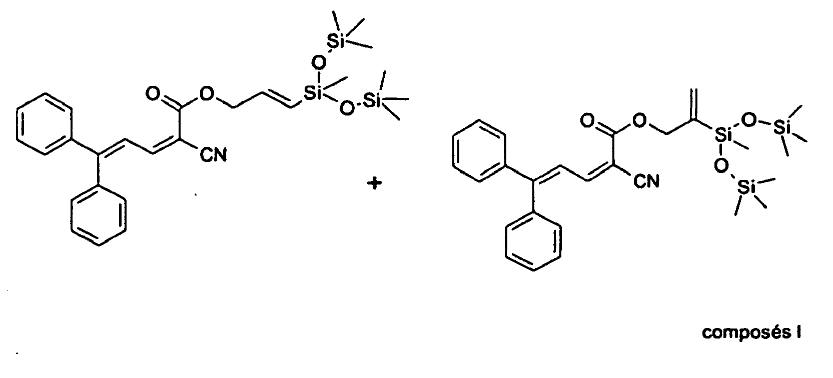

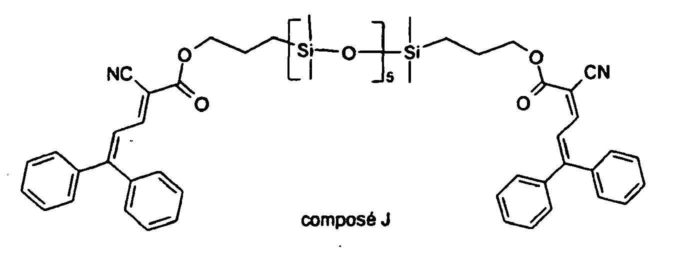

7. Utilisation d'au moins un composé diorganosiloxanique à fonction 4,4-diarylbutadiène de formule (1) ou (2) tel que défini dans l'une quelconque des revendications 1 à 6 dans la préparation une composition cosmétique ou dermatologique comme agent filtrant les radiations UV de longueurs d'onde comprises entre 320 et 400 nm.

8. Composition cosmétique ou dermatologique, destinée à la photoprotection des matières kératiniques, **caractérisée par le fait qu'**elle contient dans un support cosmétiquement acceptable au moins un composé diorganosiloxanique à fonction 4,4-diarylbutadiène répondant à la formule (1) ou (2) tel que défini dans l'une quelconque des revendications 1 à 6.

9. Composition selon la revendication 8, **caractérisée par le fait que** le composé de formule (1) ou (2) est présent dans la composition à une teneur allant de 0,01 à 20 % en poids, par rapport au poids total de la composition, de préférence allant de 0,1 % à 10 % en poids, par rapport au poids total de la composition.

10. Composition selon l'une quelconque des revendications 8 à 9, **caractérisée par le fait qu'**elle comprend en outre un ou plusieurs filtres organiques ou inorganiques complémentaires actifs dans l'UV-A et/ou UV-B.

11. Composition selon la revendication 10, **caractérisée par le fait que** lesdits filtres organiques complémentaires sont choisis les anthranilates ; les dérivés cinnamiques ; les dérivés de dibenzoylméthane ; les dérivés salicyliques, les dérivés du camphre ; les dérivés de triazine ; les dérivés de la benzophénone ; les dérivés de β,β-diphénylacrytate ; les dérivés de benzotriazole ; les dérivés de benzalmalonate ; les dérivés de benzimidazole ; les imidazolines ; les dérivés bis-benzoazolyle ; les dérivés de l'acide p-aminobenzoïque (PABA) ; les dérivés de méthylène bis-(hydroxyphényl benzotriazole) ; les dérivés de benzoxazole ; les polymères filtres et silicones filtres autres que ceux de formule (1) ou (2) ; les dimères dérivés d'α-alkylstyrène ; les 4,4-diarylbutadiènes non-siliciés et leurs mélanges.

12. Composition selon la revendication 11, **caractérisée par le fait que** lesdits filtres organiques complémentaires sont choisis parmi
Ethylhexyl Salicylate,
Ethylhexyl Methoxycinnamate
Butyl Methoxydibenzoylmethane
Octocrylene,
Phenylbenzimidazole Sulfonic Acid,
Benzophenone-3,
Benzophenone-4,
Benzophenone-5,
2-(4-diethylamino-2-hydroxybenzoyl)-benzoate de n-hexyle.
4-Methylbenzylidene camphor,
Terephthalylidene Dicamphor Sulfonic Acid,
Disodium Phenyl Dibenzimidazole Tetra-sulfonate,
2,4,6-tris-(4'-amino benzalmalonate de diisobutyle)-s-triazine
Anisotriazine,
Ethylhexyl triazone,
Diethylhexyl Butamido Triazone,
Methylène bis-Benzotriazolyl Tetramethylbutylphénol
Drometrizole Trisiloxane
Polysilicone-15
1,1-dicarboxy (2,2'-diméthyl-propyl)-4,4-diphénylbutadiène
2,4-bis-[5-1(diméthylpropyl)benzoxazol-2-yl-(4-phenyl)-imino]-6-(2-ethylhexyl)-imino-1,3,5-triazine et leurs mélanges.

13. Composition selon la revendication 10, **caractérisée par le fait que** les filtres complémentaires inorganiques sont des pigments ou des nanopigments d'oxydes métalliques, enrobés ou non.

14. Composition selon la revendication 13, **caractérisée par le fait que** lesdits pigments ou nanopigments sont choisis parmi les oxydes de titane, de zinc, de fer, de zirconium, de cérium et leurs mélanges, enrobés ou non.

15. Composition selon l'une quelconque des revendications 8 à 14, **caractérisée par le fait qu'**elle comprend en outre au moins un agent de bronzage et/ou de brunissage artificiel de la peau.

16. Composition selon l'une quelconque des revendications 8 à 15, **caractérisée par le fait qu'**elle comprend en outre au moins un adjuvant choisi parmi les corps gras, les solvants organiques, les épaississants ioniques ou non ioniques, les adoucissants, les humectants, les antioxydants, les hydratants, les desquamants, les agents anti-radicalaires, les agents antipollution, les antibactériens, les agents anti-inflammatoires, les dépigmentants, les pro-pigmentants, les opacifiants, les stabilisants, les émollients, les silicones, les agents anti-mousse, les agents répulsifs contre les insectes, les parfums, les conservateurs, les tensioactifs anioniques, cationiques, non-ioniques, zwitterioniques ou amphotères, les antagonistes de substance P, les antagonistes de substance CGRP, les charges, les pigments, les polymères, les propulseurs, les agents alcalinisants ou acidifiants

17. Composition selon l'une quelconque des revendications 8 à 16, **caractérisée par le fait qu'**il s'agit d'une composition protectrice de l'épiderme humain ou d'une composition antisolaire et qu'elle se présente sous forme d'une dispersion vésiculaire non ionique, d'une émulsion, d'une crème, d'un lait, d'un gel, d'un gel crème, d'une suspension, d'une dispersion, d'une huile, d'une poudre, d'un bâtonnet solide, d'une mousse ou d'un spray.

18. Composition selon l'une quelconque des revendications 8 à 16, **caractérisée par le fait qu'**il s'agit d'une composition de maquillage des cils, des sourcils, des ongles ou de la peau et qu'elle se présente sous forme solide ou pâteuse, anhydre ou aqueuse, d'une émulsion, d'une suspension ou d'une dispersion.

19. Composition selon l'une quelconque des revendications 8 à 16, **caractérisée par le fait qu'**il s'agit d'une composition destinée à la protection des cheveux contre les rayons ultraviolets et qu'elle se présente sous la forme d'un shampooing, d'une lotion, d'un gel, d'une émulsion, d'une dispersion vésiculaire non ionique.

20. Utilisation d'au moins un composé diorganosiloxanique de formule (1) ou (2) tel que défini dans l'une quelconque des revendications 1 à 6 dans une composition cosmétique comme agent de contrôle de la variation de la couleur de la peau due radiations UV de longueurs d'onde comprises entre 320 et 400 nm.

21. Utilisation d'au moins un composé diorganosiloxanique de formule (1) ou (2) tel que défini dans l'une quelconque des revendications 1 à 6 comme agent photostabilisant de polymères synthétiques ou de verres.

## Claims

1. Diorganosiloxane compound containing a 4,4-diarylbutadiene function, in isolated form or in the form of a mixture, corresponding to one of the general formulae (1) or (2) below: 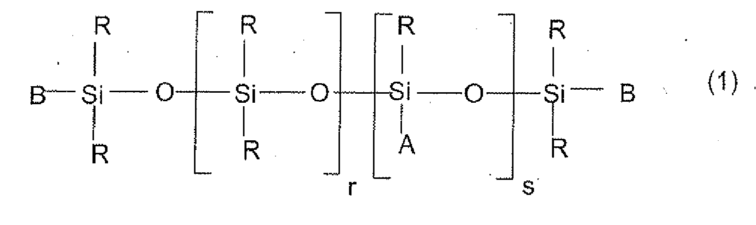 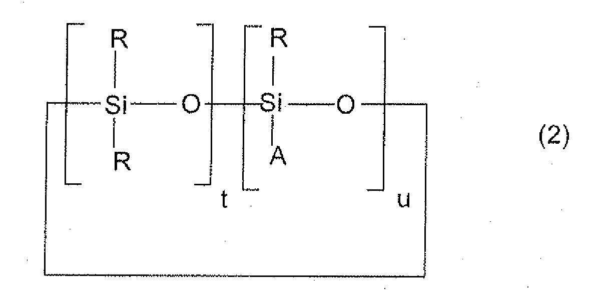 in which:
- A denotes a radical of formula (3) below: 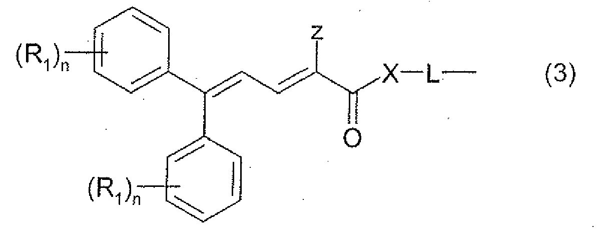
in which:
- the radicals R₁, which may be identical or different, denote a hydroxyl radical; a saturated or unsaturated, linear or branched C₁-C₁₀ alkyl radical; a saturated or unsaturated, linear or branched C₁-C₁₀ alkoxy radical, two adjacent groups R₁ together possibly forming a C₁-C₃ alkylenedioxy ring;
- n is an integer ranging from 0 to 2;
- the radical Z denotes hydrogen; -(C=O)OR₂, -(C=O)R₃,
- (C=O)NR₄R₅, -SO₂R₆, -CN or -(C=O)-X-L-;
- X denotes -O- or -NR₄-;
- L is a divalent radical that allows the attachment of the radical A to the silicone chain corresponding to one of the formulae (a), (a') or (a") below: 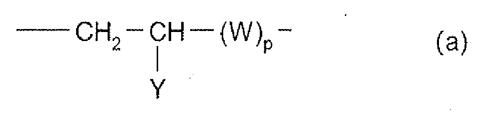
- CH=CH-(W)ₚ- (a') 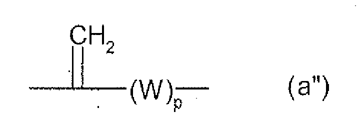
in which:
- W is a saturated or unsaturated, linear or branched C₁-C₆ alkylene radical, optionally substituted with a hydroxyl radical or a saturated or unsaturated, linear or branched C₁-C₈ alkyl radical,
- Y represents a hydrogen atom; a hydroxyl radical or a saturated or unsaturated, linear or branched C₁-C₈ alkyl radical,
- p is 0 or 1,
- the radicals R, which may be identical or different, denote a saturated or unsaturated, linear or branched C₁-C₂₀ alkyl group; a phenyl group; a 3,3,3-trifluoropropyl group or a trimethylsilyloxy group; at least 80% in numerical terms of the radicals R being methyl,
- B, which may be identical or different, are chosen from the radicals R and the radical A,
- r is a number ranging from 0 to 50,
- s is a number ranging from 0 to 20 and if s is 0, at least one of the two symbols B is A,
- u is a number ranging from 1 to 6 inclusive,
- t is a number ranging from 0 to 10,
- t+u is greater than or equal to 3,
- the radical R₂ denotes hydrogen; a saturated or unsaturated, linear or branched C₁-C₂₀ alkyl radical,
- the radical R₃ denotes a saturated or unsaturated, linear or branched C₁-C₂₀ alkyl radical; a C₆-C₁₂ aryl,
- the radicals R₄ and R₅, which may be identical or different, represent hydrogen; a saturated or unsaturated, linear or branched C₁-C₂₀ alkyl radical,
- the radical R₆ is a saturated or unsaturated, linear or branched C₁-C₂₀ alkyl radical; a C₆-C₁₂ aryl,
with the proviso that when two groups (C=O)-X-L- are present, L is linked to only one group A or B of formula (1) or (2): i.e. s = 1 with B being other than A or s = 0 and only one B is A for formula (1) and u = 1 for formula (2).

2. Diorganosiloxane compound according to Claim 1, satisfying at least one, and even more preferably all, of the following characteristics:
- the radical Z denotes -(C=O)R₃, -CN or -(C=O)-X-L-,
- R₃ is C₁-C₄ alkyl and even more preferably ethyl,
- R is C₁-C₄ alkyl and even more preferably methyl,
- B is C₁-C₄ alkyl and even more preferably methyl (in the case where the compound is of formula (1)),
- r ranges from 0 to 10 inclusive; s ranges from 0 to 6 inclusive (in the case where the compound is of formula (1)),
- t+u ranges from 3 to 5 (in the case where the compound is of formula (2)),
- n is 0,
- W denotes CH₂,
- p is 1,
- Y denotes H or CH₃.

3. Diorganosiloxane compound containing a 4,4-diarylbutadiene function of formula (1) according to Claim 1 or 2, chosen from the group consisting of compounds A, B, C, D and E below:

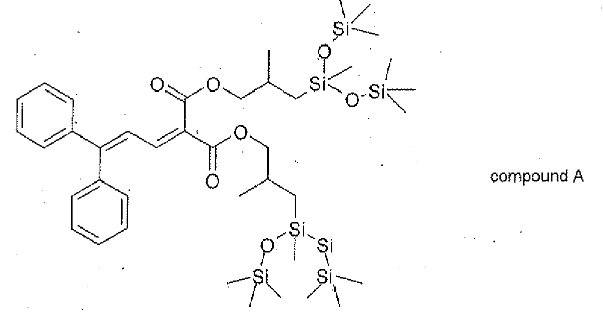

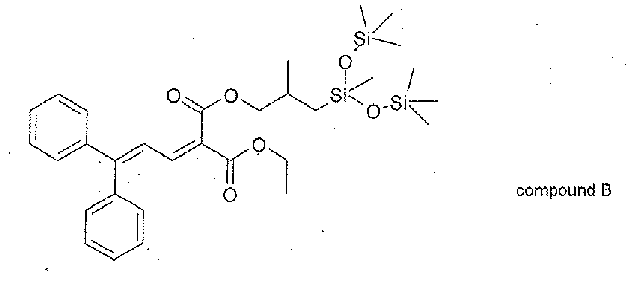

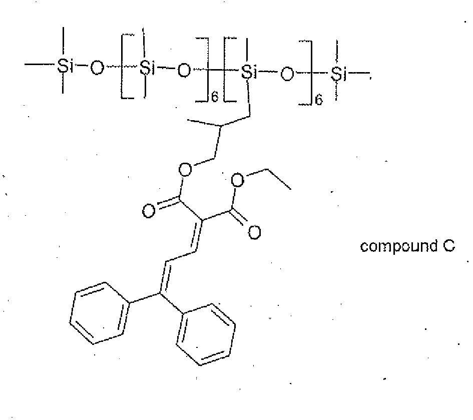

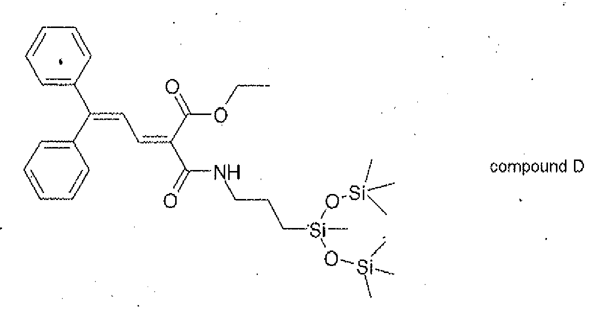

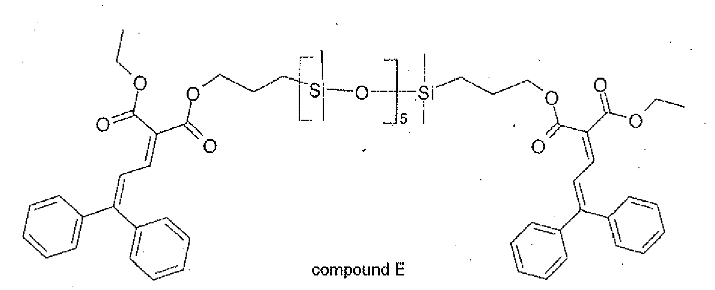

4. Diorganosiloxane compound containing a 4,4-diarylbutadiene function of formula (1) or (2) according to Claim 1 or 2, in which Z denotes CN.

5. Diorganosiloxane compound containing a 4,4-diarylbutadiene function of formula (1) or (2) according to Claim 4, satisfying at least one, and even more preferably all, of the following characteristics:
- R is C₁-C₄ alkyl and even more preferably methyl,
- B is C₁-C₄ alkyl and even more preferably methyl (in the case where the compound is of formula (1)),
- r ranges from 0 to 10 inclusive; s ranges from 0 to 6 inclusive (in the case where the compound is of formula (1)),
- t+u ranges from 3 to 5 (in the case where the compound is of formula (2)),
- n is 0,
- W denotes CH₂,
- p is 1,
- Y denotes H or CH₃.

6. Diorganosiloxane compound containing a 4,4-diarylbutadiene function according to Claim 1 or 2, chosen from the group consisting of compounds F, G, H, I and J below:

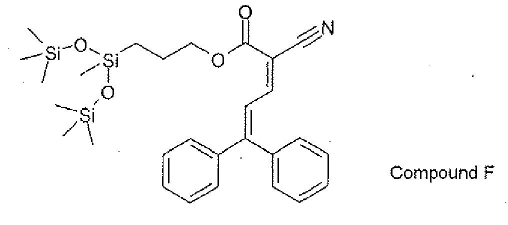

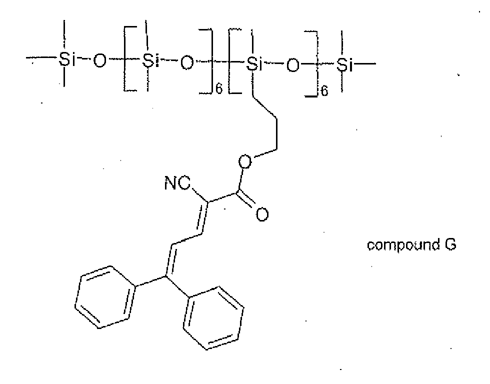

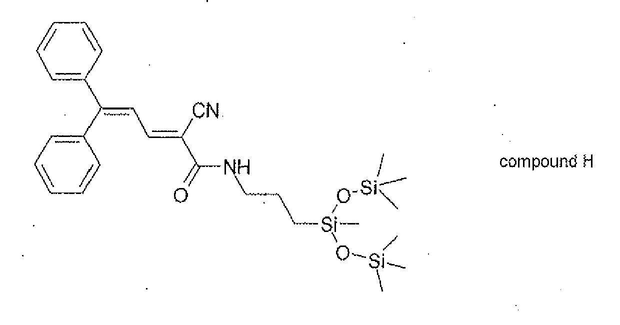

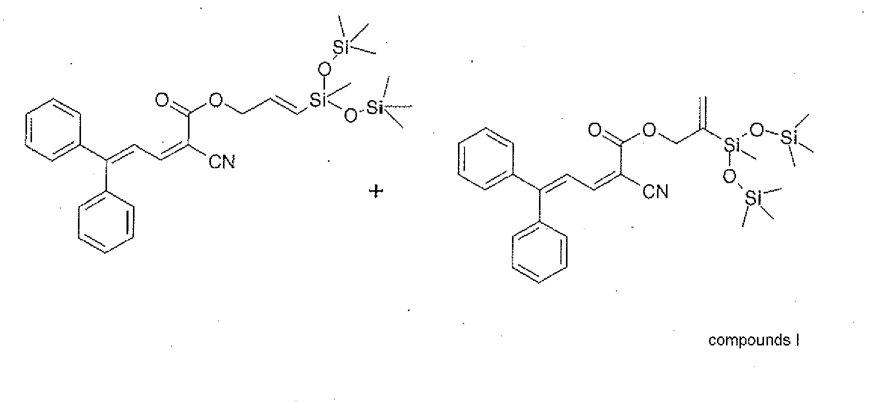

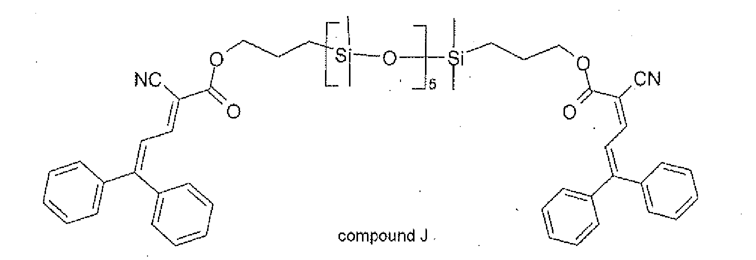

7. Use of at least one diorganosiloxane compound containing a 4,4-diarylbutadiene function of formula (1) or (2) as defined in any one of Claims 1 to 6, in the preparation of a cosmetic or dermatological composition, as an agent for screening out UV radiation with wavelengths of between 320 and 400 nm.

8. Cosmetic or dermatological composition for photoprotecting keratin materials, **characterized in that** it contains, in a cosmetically acceptable support, at least one diorganosiloxane compound containing a 4,4-diarylbutadiene function corresponding to formula (1) or (2) as defined in any one of Claims 1 to 6.

9. Composition according to Claim 8, **characterized in that** the compound of formula (1) or (2) is present in the composition in a content ranging from 0.01% to 20% by weight relative to the total weight of the composition, and preferably ranging from 0.1% to 10% by weight relative to the total weight of the composition.

10. Composition according to either of Claims 8 and 9, **characterized in that** it also comprises one or more additional UV-A-active and/or UV-B-active organic or mineral screening agents.

11. Composition according to Claim 10, **characterized in that** the said additional organic screening agents are chosen from anthranilates; cinnamic derivatives; dibenzoylmethane derivatives; salicylic derivatives; camphor derivatives; triazine derivatives; benzophenone derivatives; β,β-diphenylacrylate derivatives; benzotriazole derivatives; benzalmalonate derivatives; benzimidazole derivatives; imidazolines; bis-benzazolyl derivatives; p-aminobenzoic acid (PABA) derivatives; methylenebis(hydroxyphenylbenzotriazole) derivatives; benzoxazole derivatives; screening polymers and screening silicones other than those of formula (1) or (2); α-alkylstyrene-based dimers; non-siliceous 4,4-diarylbutadienes, and mixtures thereof.

12. Composition according to Claim 11, **characterized in that** the said additional organic screening agents are chosen from:
Ethylhexyl salicylate,
Ethylhexyl methoxycinnamate,
Butyl methoxydibenzoylmethane,
Octocrylene,
Phenylbenzimidazolesulfonic acid,
Benzophenone-3,
Benzophenone-4,
Benzophenone-5,
n-Hexyl 2-(4-diethylamino-2-hydroxybenzoyl)benzoate,
4-Methylbenzylidenecamphor,
Terephthalylidenedicamphorsulfonic acid,
Disodium phenyldibenzimidazoletetrasulfonate,
2,4,6-Tris(diisobutyl 4'-aminobenzalmalonate)-s-triazine,
Anisotriazine,
Ethylhexyltriazone,
Diethylhexylbutamidotriazone,
Methylenebis(benzotriazolyl)tetramethylbutylphenol,
Drometrizole trisiloxane,
Polysilicone-15
1,1-Dicarboxy(2,2'-dimethylpropyl)-4,4-diphenylbutadiene,
2,4-Bis[5-1(dimethylpropyl)benzoxazol-2-yl(4-phenyl)imino]-6-(2-ethylhexyl)imino-1,3,5-triazine
and mixtures thereof.

13. Composition according to Claim 10, **characterized in that** the additional mineral screening agents are coated or uncoated metal oxide pigments or nanopigments.

14. Composition according to Claim 13, **characterized in that** the said pigments or nanopigments are chosen from coated or uncoated titanium oxide, zinc oxide, iron oxide, zirconium oxide and cerium oxide, and mixtures thereof.

15. Composition according to any one of Claims 8 to 14, **characterized in that** it also comprises at least one agent for artificially tanning and/or browning the skin.

16. Composition according to any one of Claims 8 to 15, **characterized in that** it also comprises at least one adjuvant chosen from fatty substances, organic solvents, ionic or nonionic thickeners, softeners, humectants, antioxidants, moisturizers, desquamating agents, free-radical scavengers, antipollution agents, antibacterial agents, anti-inflammatory agents, depigmenting agents, propigmenting agents, opacifiers, stabilizers, emollients, silicones, antifoams, insect repellents, fragrances, preserving agents, anionic, cationic, nonionic, zwitterionic or amphoteric surfactants, substance P antagonists, substance CGRP antagonists, fillers, pigments, polymers, propellants, acidifying or basifying agents.

17. Composition according to any one of Claims 8 to 16, **characterized in that** it is a composition for protecting the human epidermis or an antisun composition and **in that** it is in the form of a nonionic vesicular dispersion, an emulsion, a cream, a milk, a gel, a cream-gel, a suspension, a dispersion, an oil, a powder, a solid tube, a mousse or a spray.

18. Composition according to any one of Claims 8 to 16, **characterized in that** it is a composition for making up the eyelashes, the eyebrows, the nails or the skin and **in that** it is in solid or pasty, anhydrous or aqueous form or in the form of an emulsion, a suspension or a dispersion.

19. Composition according to any one of Claims 8 to 16, **characterized in that** it is a composition for protecting the hair against ultraviolet rays and **in that** it is in the form of a shampoo, a lotion, a gel, an emulsion or a nonionic vesicular dispersion.

20. Use of at least one diorganosiloxane compound of formula (1) or (2) as defined in any one of Claims 1 to 6, in a cosmetic composition as an agent for controlling the variation in the colour of the skin caused by UV radiation with wavelengths of between 320 and 400 nm.

21. Use of at least one diorganosiloxane compound of formula (1) or (2) as defined in any one of Claims 1 to 6, as an agent for photostabilizing synthetic polymers or lenses.

## Patentansprüche

1. Diorganosiloxan-Verbindung mit 4,4-Diarylbutadien-Funktion in isolierter Form oder in Form von Gemischen entsprechend einer der nachstehenden allgemeinen Formeln (1) oder (2): 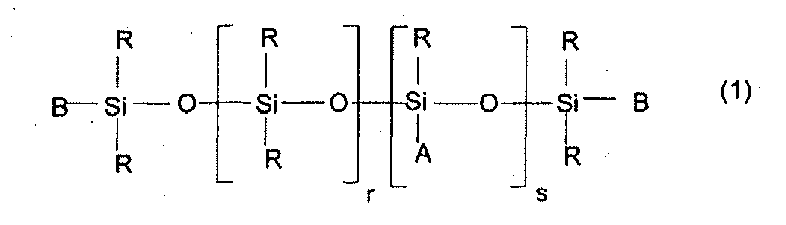 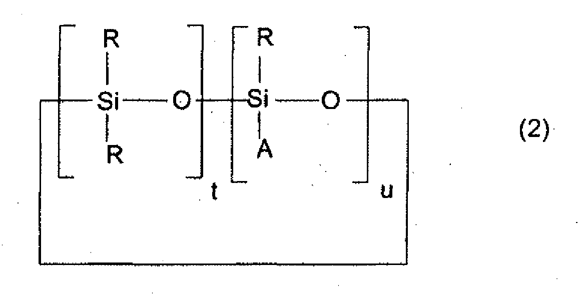 worin
- A eine Gruppe der nachstehenden Formel (3) darstellt, 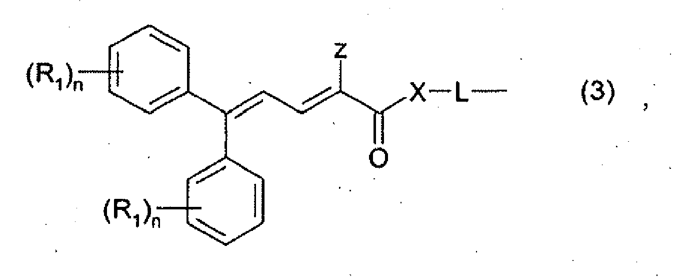
in der bedeuten:
- die Gruppen R1, die gleich oder verschieden sind, eine Hydroxylgruppe, geradkettiges oder verzweigtes, gesättigtes oder ungesättigtes C₁-C₁₀-Alkyl, geradkettiges oder verzweigtes, gesättigtes oder ungesättigtes C₁-C₁₀-Alkoxy, wobei zwei benachbarte Gruppen R₁ zusammen einen C₁-C₃-Alkylendioxy-Ring bilden können;
- n eine ganze Zahl von 0 bis 2;
- Z Wasserstoff; -(C=O)OR₂, -(C=O)R₃, -(C=O)NR₄R₅, -SO₂R₆, -CN oder -(C=O)-X-L-,
- X -O- oder -NR4-;
- L eine zweiwertige Gruppe, welche die Verknüpfung der Gruppe A mit der Siliconkette erlaubt und einer der nachstehenden Formeln (a), (a') oder (a") entspricht, 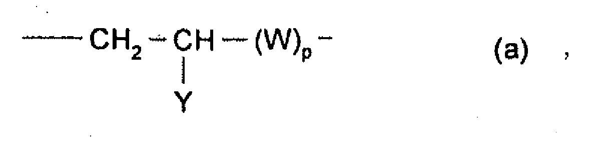
-CH=CH-(W)ₚ- (a'), 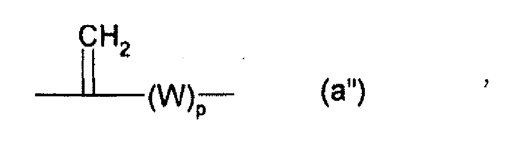
in denen bedeuten:
- W eine geradkettige oder verzweigte, gesättigte oder ungesättigte C₁-C₆ Alkylgruppe, die gegebenenfalls mit einer Hydroxylgruppe oder einer geradkettigen oder verzweigten, gesättigten oder ungesättigten C₁-C₈ Alkylgruppe substituiert ist,
- Y ein Wasserstoffatom; eine Hydroxylgruppe oder eine geradkettige oder verzweigte, gesättigte oder ungesättigte C₁-C₈-Alkylgruppe,
- p 0 oder 1,
- die Gruppen R, die gleich oder verschieden sind, geradkettiges oder verzweigtes, gesättigtes oder ungesättigtes C₁-C₂₀-Alkyl, eine Phenylgruppe, 3,3,3-Trifluorpropyl oder Trimethylsilyloxy bedeuten, wobei mindestens 80 % der Gruppen R, bezogen auf ihre Anzahl, Methyl darstellen,
- B, die gleich oder verschieden sind, unter den Gruppen R und der Gruppe A ausgewählt sind,
- r eine Zahl von 0 bis 50 ist,
- s eine Zahl von 0 bis 20 ist,
wobei, wenn s gleich Null ist, mindestens eine der beiden Gruppen B gleich A ist,
- u eine ganze Zahl von 1 bis einschließlich 6 ist,
- t eine Zahl von 0 bis 10 ist,
- wobei t + u mindestens gleich 3 ist,
wobei
- der Substituent R₂ Wasserstoff oder geradkettiges oder verzweigtes, gesättigtes oder ungesättigtes C₁-C₂₀-Alkyl bedeutet,
- die Gruppe R₃ geradkettiges oder verzweigtes, gesättigtes oder ungesättigtes C₁- C₂₀-Alkyl bedeutet,
- die Substituenten R₄ und R₅, die gleich oder verschieden sind, Wasserstoff oder geradkettiges oder verzweigtes, gesättigtes oder ungesättigtes C₁-C₂₀-Alkyl bedeuten,
- die Gruppe R₆ geradkettiges oder verzweigtes, gesättigtes
oder ungesättigtes C₁-C₂₀-Alkyl oder C₆-C₁₂-Aryl bedeutet, mit der Maßgabe, dass, wenn zwei Gruppen (C=O)-X-L-vorliegen, L nur an eine einzige Gruppe A oder B der Formeln (1) oder (2) gebunden ist, was bedeutet: s = 1, wobei B von A verschieden ist, oder s = 0, wobei ein einziges B gleich A bei Formel (1) und u = 1 bei Formel (2) ist.

2. Diorganosiloxan-Verbindung nach Anspruch 1, die mindestens eine der nachstehenden Eigenschaften und noch bevorzugter sämtliche nachstehenden Eigenschaften aufweist:
- Der Substituent Z bezeichnet -(C=O)R₃, -CN oder -(C=O)-X-L-,
- R₃ ist C₁-C₄-Alkyl und noch bevorzugter Ethyl,
- R ist C₁-C₄-Alkyl und noch bevorzugter Methyl,
- B ist C₁-C₄-Alkyl und noch bevorzugter Methyl (wenn die Verbindung die Formel (1) besitzt),
- r liegt im Bereich von 0 bis einschließlich 10,
- s liegt im Bereich von 0 bis einschließlich 6 (wenn die Verbindung die Formel (1) besitzt),
- t+u liegt im Bereich von 3 bis 5 (wenn die Verbindung die Formel (2) besitzt),
- n = 0,
- W bezeichnet CH₂,
- p = 1,
- Y bezeichnet H oder CH₃.

3. Diorganosiloxan-Verbindung mit 4,4-Diarylbutadien-Funktion der Formel (1) nach Anspruch 1 oder 2, die aus der Gruppe ausgewählt ist, die aus den nachstehenden Verbindungen A, B, C, D und E besteht:

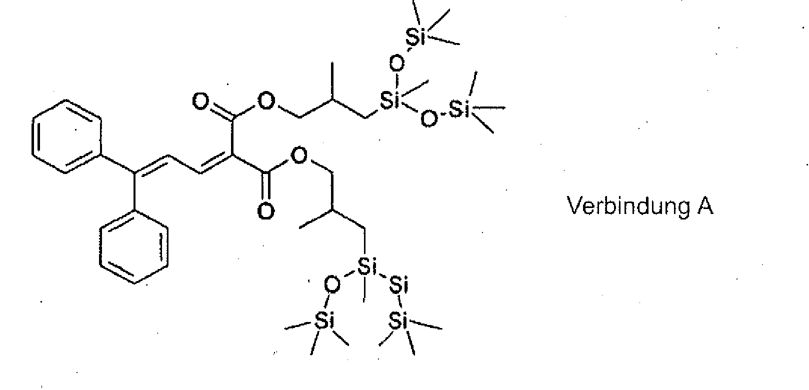

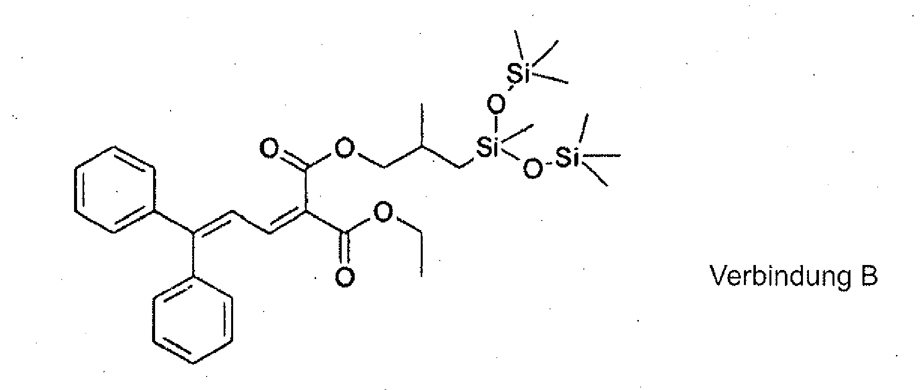

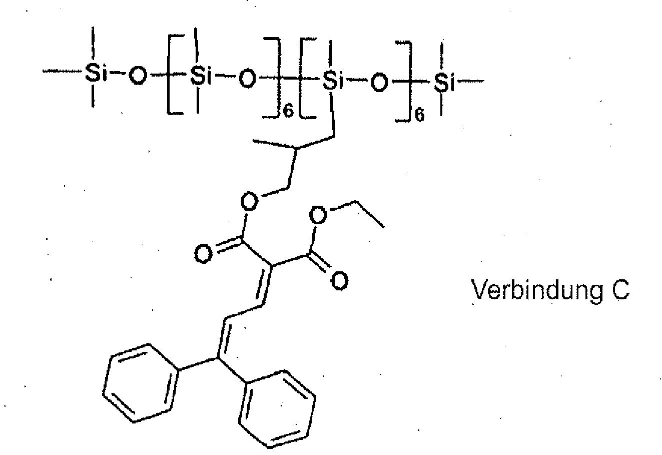

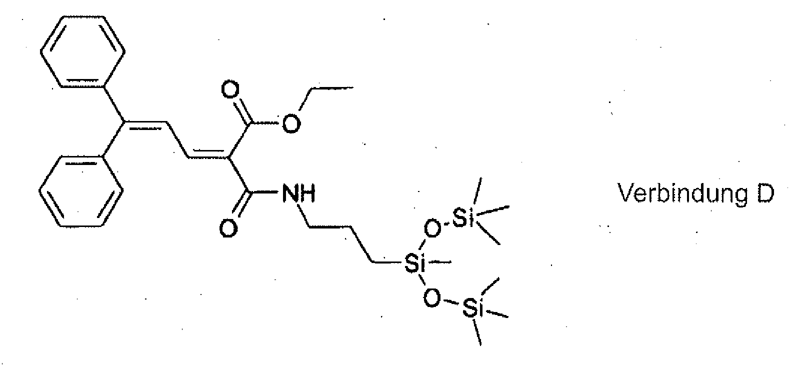

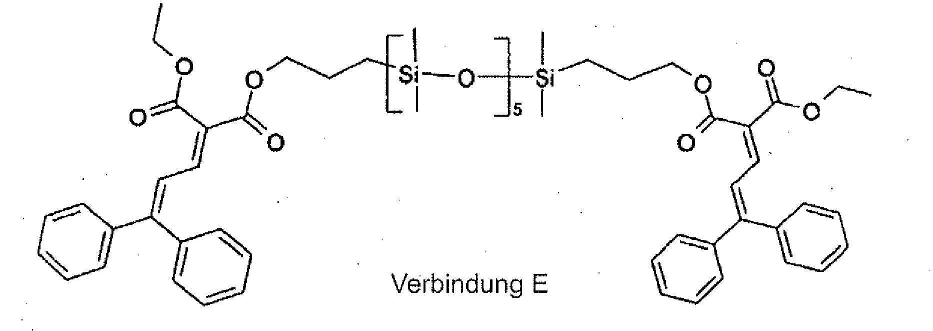

4. Diorganosiloxan-Verbindung mit 4,4-Diarylbutadien-Funktion der Formel (1) oder (2) nach Anspruch 1 oder 2, worin Z -CN bezeichnet.

5. Diorganosiloxan-Verbindung mit 4,4-Diarylbutadien-Funktion der Formel (1) oder (2) nach Anspruch 4, die mindestens eine der nachstehenden Eigenschaften und noch bevorzugter sämtliche nachstehenden Eigenschaften aufweist:
- R ist C₁-C₄-Alkyl und noch bevorzugter Methyl,
- B ist C₁-C₄-Alkyl und noch bevorzugter Methyl (wenn die Verbindung die Formel (1) besitzt),
- r liegt im Bereich von 0 bis einschließlich 10; s liegt im Bereich von 0 bis einschließlich 6 (wenn die Verbindung die Formel (1) besitzt),
- t + u liegt im Bereich von 3 bis 5 (wenn die Verbindung die Formel (2) besitzt),
- n = 0,
- W bezeichnet CH₂,
- p = 1,
- Y bezeichnet H oder CH₃.

6. Diorganosiloxan-Verbindung mit 4,4-Diarylbutadien-Funktion nach Anspruch 1 oder 2, die aus der Gruppe ausgewählt ist, die aus den nachstehenden Verbindungen F, G, H, I, J besteht:

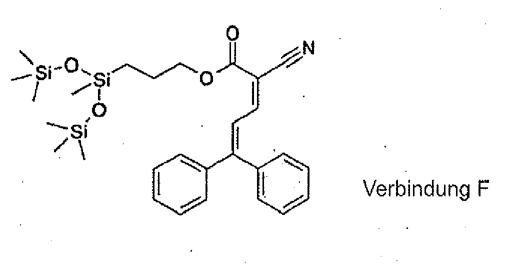

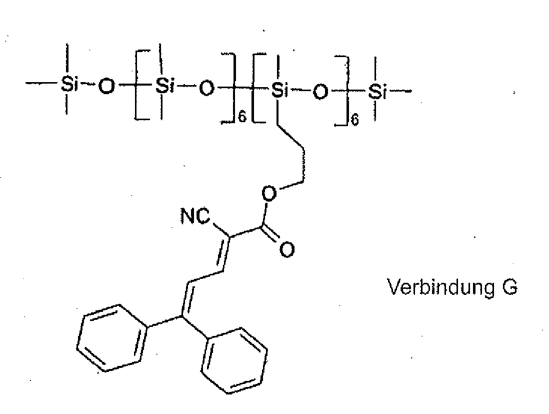

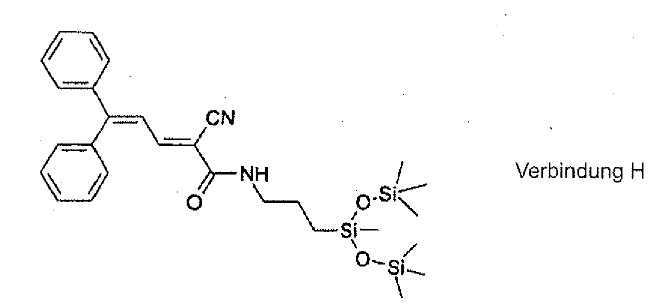

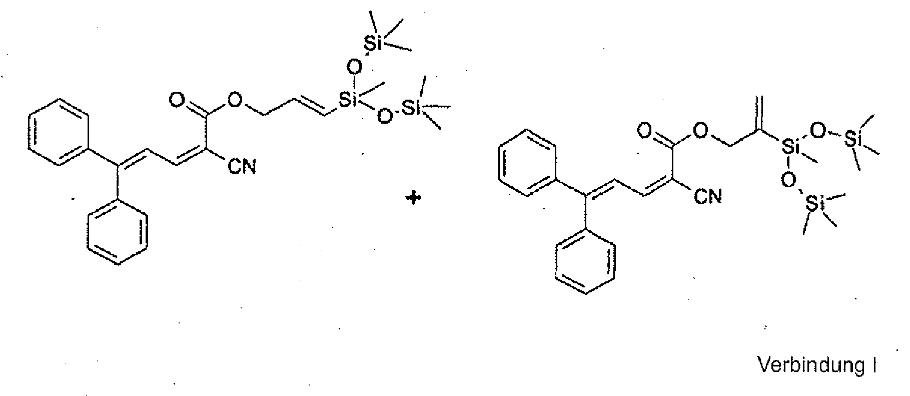

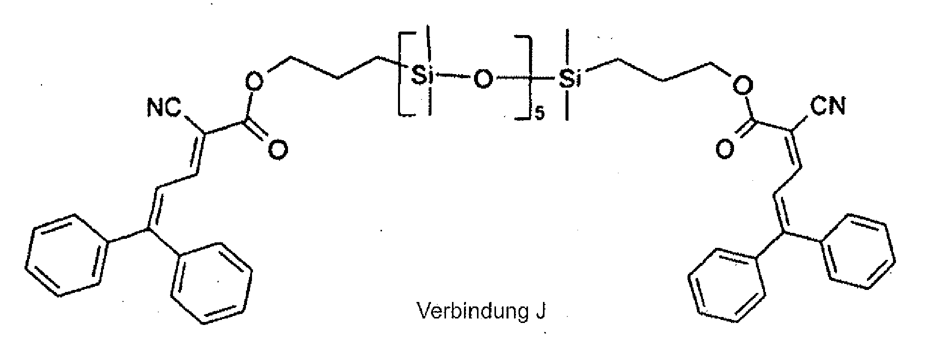

7. Verwendung mindestens einer Diorganosiloxan-Verbindung mit 4,4-Diarylbutadien-Funktion der Formel (1) oder (2) nach einem der Ansprüche 1 bis 6 bei der Herstellung einer kosmetischen oder dermatologischen Zusammensetzung als Filter für UV-Strahlung mit Wellenlängen im Bereich von 320 bis 400 nm.

8. Kosmetische oder dermatologische Zusammensetzung zum Lichtschutz von Keratinmaterialien, **dadurch gekennzeichnet, dass** sie in einem kosmetisch akzeptablen Träger mindestens eine Diorganosiloxan-Verbindung mit 4,4-Diarylbutadien-Funktion der Formel (1) oder (2) nach einem der Ansprüche 1 bis 6 enthält.

9. Zusammensetzung nach Anspruch 8, **dadurch gekennzeichnet, dass** die Verbindung der Formel (1) oder (2) in der Zusammensetzung in einem Mengenanteil von 0,01 bis 20 Gew.-%, bezogen auf das Gesamtgewicht der Zusammensetzung, und vorzugsweise in einem Mengenanteil von 0,1 bis 10 Gew.-% vorliegt, bezogen auf das Gesamtgewicht der Zusammensetzung.

10. Zusammensetzung nach Anspruch 8 oder 9, **dadurch gekennzeichnet, dass** sie ferner ein oder mehrere zusätzliche organische oder anorganische Filter enthält, die im UV-A-Bereich und/oder im UV-B-Bereich wirksam sind.

11. Zusammensetzung nach Anspruch 10, **dadurch gekennzeichnet, dass** die zusätzlichen organischen Filter ausgewählt sind unter: Anthranilaten; Zimtsäurederivaten; Derivaten von Dibenzoylmethan; Salicylsäurederivaten, Campherderivaten; Triazinderivaten; Derivaten von Benzophenon; Derivaten von β,β-Diphenylacrylat, Benzotriazolderivaten; Benzalmalonat-Derivaten; Benzimidazolderivaten, Imidazolinen; Bis-Benzoazolylderivaten; Derivaten von p-Aminobenzosäure (PABA) ; Derivaten von Methylen-bis(hydroxyphenylbenzotriazol); Benzoxazolderivaten; polymeren Filtern und Silicon-Filtern, die von den Filtern der Formel (1) oder (2) verschieden sind; von α-Alkylstyrol abgeleiteten Dimeren; 4,4-Diarylbutadienen, die kein Silicium enthalten, sowie Gemischen dieser Verbindungen.

12. Zusammensetzung nach Anspruch 11, **dadurch gekennzeichnet, dass** die zusätzlichen organischen Filter ausgewählt sind unter
Ethylhexyl Salicylate,
Ethylhexyl Methoxycinnamate
Butyl Methoxydibenzoylmethane
Octocrylene,
Phenylbenzimidazole Sulfonic Acid,
Benzophenone-3,
Benzophenone-4,
Benzophenone-5,
2-(4-Diethylamino-2-hydroxybenzoyl)-benzoesäure-n-hexylester,
4-Methylbenzylidene Camphor,
Terephthalylidene Dicamphor Sulfonic Acid,
Disodium Phenyl Dibenzimidazole Tetra-sulfonate,
2,4,6-Tris(4'-aminobenzalmalonsäurediisobutylester)-s-triazin,
Anisotriazine,
Ethylhexyl Triazone,
Diethylhexyl Butamido Triazone,
Methylene bis-Benzotriazolyl Tetramethylbutylphenol,
Drometrizole Trisiloxane,
Polysilicone-15,
1,1-Dicarboxy-(2,2'-dimethylpropyl)-4,4-diphenylbutadien,
2,4-Bis[5-1(dimethylpropyl)-benzoxazol-2-yl-(4-phenyl)-imino]-6-(2-ethylhexyl)-imino-1,3,5-triazin
sowie Gemischen dieser Verbindungen.

13. Zusammensetzung nach Anspruch 10, **dadurch gekennzeichnet, dass** die zusätzlichen anorganischen Filter Pigmente oder Nanopigmente von Metalloxiden sind, die gegebenenfalls ummantelt sind.

14. Zusammensetzung nach Anspruch 13, **dadurch gekennzeichnet, dass** die Pigmente oder Nanopigmente ausgewählt sind unter den Oxiden von Titan, Zink, Eisen, Zirkonium, Cer sowie Gemischen davon, wobei die Pigmente oder Nanopigmente gegebenenfalls ummantelt sind.

15. Zusammensetzung nach einem der Ansprüche 8 bis 14, **dadurch gekennzeichnet, dass** sie ferner mindestens ein Mittel zur künstlichen Bräunung und/oder ein Mittel zur künstlichen Braunfärbung der Haut enthält.

16. Zusammensetzung nach einem der Ansprüche 8 bis 15, **dadurch gekennzeichnet, dass** sie ferner mindestens einen Hilfsstoff enthält, der ausgewählt ist unter Fettstoffen, organischen Lösungsmitteln, ionischen oder nichtionischen Verdickungsmitteln, reizlindernden Mitteln, Befeuchtungsmitteln, Antioxidationsmitteln, Hydratisierungsmitteln, Desquamationsmitteln, Mitteln gegen freie Radikale, Mitteln gegen Verschmutzung, antibakteriellen Mitteln, entzündungshemmenden Mitteln, Depigmentierungsmitteln, die Pigmentierung fördernden Mitteln, Trübungsmitteln, Stabilisierungsmitteln, Emollientien, Siliconen, Antischaummitteln, Insektenrepellents, Parfums, Konservierungsmitteln, anionischen, kationischen, nicht-ionischen, zwitterionischen oder amphoteren Tensiden, Antagonisten der Substanz P, Antagonisten der Substanz CGRP, Füllstoffen, Pigmenten, Polymeren, Treibmitteln, Mitteln zum Alkalischmachen oder Mitteln zum Ansäuern.

17. Zusammensetzung nach einem der Ansprüche 8 bis 16, **dadurch gekennzeichnet, dass** es sich um eine Zusammensetzung zum Schutz der menschlichen Epidermis oder eine Zusammensetzung zum Sonnenschutz handelt und sie vorliegt in Form einer nichtionischen Vesikeldispersion, einer Emulsion, einer Creme, einer Milch, eines Gels, einer Gelcreme, einer Suspension, einer Dispersion, eines Öls, eines Puders, eines festen Sticks, eines Schaums oder eines Sprays.

18. Zusammensetzung nach einem der Ansprüche 8 bis 16, **dadurch gekennzeichnet, dass** es sich um eine Zusammensetzung zum Schminken der Wimpern, der Augenbrauen, der Nägel oder der Haut handelt und sie in wasserfreier oder wässeriger, fester oder pastoser Form, in Form einer Emulsion, in Form einer Suspension oder in Form einer Dispersion vorliegt.

19. Zusammensetzung nach einem der Ansprüche 8 bis 16, **dadurch gekennzeichnet, dass** es sich um eine Zusammensetzung zum Schutz der Haare gegen Ultraviolettstrahlung handelt und sie in Form eines Shampoos, einer Lotion, eines Gels, einer Emulsion oder einer nichtionischen Vesikeldispersion vorliegt.

20. Verwendung mindestens einer Diorganosiloxan-Verbindung der Formel (1) oder (2) nach einem der Ansprüche 1 bis 6 in einer kosmetischen Zusammensetzung als Mittel zur Kontrolle der Farbänderung der Haut durch UV-Strahlung im Wellenlängenbereich von 320 bis 400 nm.

21. Verwendung mindestens einer Diorganosiloxan-Verbindung der Formel (1) oder (2) nach einem der Ansprüche 1 bis 6 als Mittel zur Lichtstabilisierung von synthetischen Polymeren oder von Gläsern.
